# EUROPEAN PATENT APPLICATION

(11) **EP 4 574 169 A1**
(43) Date of publication of application: **25.06.2025**
(21) Application number: 23854891.1
(22) Date of filing: 15.08.2023
(51) Int. Cl.: A61K 39/215, A61K 35/763, A61P 31/14, C12N 15/50, C12N 15/869, C12P 21/02

(54) **PHARMACEUTICAL COMPOSITION CONTAINING HERPES SIMPLEX VIRUS VECTOR**

(30) Priority: 16.08.2022 JP 2022129775
(71) Applicant: Todo, Tomoki, Koto-ku Tokyo 135-0034 (JP)
(72) Inventor: Todo, Tomoki, Koto-ku Tokyo 135-0034 (JP)
(74) Representative: Hoffmann Eitle
(86) International application number: PCT/JP2023/029503
(87) International publication number: WO 2024/038857

(57) **Abstract**

An object is to provide a virus vector that can be used as a vaccine. A pharmaceutical composition for producing an exogenous polypeptide and inducing an immune response thereto, comprising a herpes simplex virus vector containing a region encoding the exogenous polypeptide and having two or more characteristics selected from the following characteristics (a) to (c):
(a) the ICP6 gene is deleted or inactivated,
(b) the γ34.5 gene is deleted or inactivated, and
(c) the α47 gene is deleted or inactivated.

## Description

### Technical Field

The present invention relates to a herpes simplex virus vector. The herpes simplex virus vector of the present invention can be used as a vaccine.

### Background Art

With the spread of novel coronavirus infection (COVID-19) beginning around the end of 2019, vaccine development is becoming increasingly important. In particular, messenger RNA (mRNA) vaccines and viral vector vaccines, with which partial genetic information of a virus is administered to the human body, are attracting attention as vaccines with a different mechanism from the vaccines of the conventional type for administering a part of a virus protein.

Meanwhile, oncolytic virus therapy is attracting attention as the fourth treatment method for cancers, following surgery, chemotherapy, and radiotherapy. Herpes simplex virus type 1 (HSV-1) is one of the viruses used in oncolytic virus therapies for cancers, and recombinant oncolytic viruses have been developed by introducing artificial genetic modifications into the genome of this virus to enhance safety and therapeutic effect (Patent documents 1 to 4, and Non-patent document 1 to 5).

### Prior Art References

### Patent documents

Patent document 1: Japanese Patent No. 4212897
Patent document 2: Japanese Patent No. 4921669
Patent document 3: WO2011/101912
Patent document 4: WO2019/189643

### Non-patent documents

Non-patent document 1: Todo, T. et al. (2000). Molecular Therapy, 2, 588-595
Non-patent document 2: Markert, J. M. et al. (2000). Gene Therapy, 7, 867-874
Non-patent document 3: Martuza, R. L. et al. (2000). Journal of Clinical Investigation, 105, 841-846
Non-patent document 4: Fukuhara, H. et al. (2005). Cancer Res, 65 (23), 10663-10668
Non-patent document 5: Fukuhara, H. et al. (2023). Commun Med (London), 3 (1), 40. https://www.nature.com/articles/s43856-023-00270-4 (unpublished on the priority date of this application)

### Summary of Invention

### Problem to be solved by the invention

Use of viruses of the genus *Simplexvirus* as vaccine virus vectors has not been explored so far.

### Means for solving the problem

Viruses to be used for gene transfer as vaccines desirably satisfy the following conditions: 1) a large exogenous gene can be inserted into the viral genome, 2) an exogenous gene introduced functions as an epigenome without being incorporated into the human genome, 3) the same effect as that obtained by expressing multiple types of exogenous genes can be obtained with a mixture of multiple types of viruses that each express a single type of exogenous gene, and 4) even when they are repeatedly administered, the same expression effect can be achieved every time without being inhibited by immunity to the viruses themselves.

The inventor of the present invention has been engaged in the clinical development of a third-generation herpes virus G47Δ for cancer therapy, obtained from HSV-1 by modifications of three viral genes, recently found that G47Δ incorporated with a cDNA encoding a novel coronavirus spike protein can be effectively used as a vaccine virus, and accomplished the present invention.

The present invention provides the followings.
[1] A pharmaceutical composition for producing an exogenous polypeptide and inducing an immune response thereto, the composition comprising a herpes simplex virus vector containing a region encoding the exogenous polypeptide and having two or more of characteristics selected from the following characteristics (a) to (c):
   (a) the ICP6 gene is deleted or inactivated,
   (b) the γ34.5 gene is deleted or inactivated, and
   (c) the α47 gene is deleted or inactivated.
[2] The pharmaceutical composition according to 1, wherein the herpes simplex virus vector has all the characteristics (a) to (c).
[3] The pharmaceutical composition according to claim 2, wherein the region encoding the exogenous polypeptide is inserted into a deleted or inactivated site of the ICP6 gene.
[4] The pharmaceutical composition according to any one of 1 to 3, which is for producing the exogenous polypeptide in noncancerous cells.
[5] The pharmaceutical composition according to any one of 1 to 4, which is an agent for subcutaneous or intramuscular injection.
[6] The pharmaceutical composition according to any one of 1 to 5, wherein the exogenous polypeptide contains all or a part of a pathogen-derived protein.
[7] The pharmaceutical composition according to any one of 1 to 6, wherein the exogenous polypeptide contains all or a part of a spike protein derived from SARS-CoV-2.
   The present invention also provides the followings.
[8] A pharmaceutical composition for producing an exogenous polypeptide in noncancerous cells, the composition comprising a herpes simplex virus vector containing a region encoding the exogenous polypeptide and having two or more of characteristics selected from the following characteristics (a) to (c):
   (a) the ICP6 gene is deleted or inactivated,
   (b) the γ34.5 gene is deleted or inactivated, and
   (c) the α47 gene is deleted or inactivated.
[9] The pharmaceutical composition according to 8, wherein the herpes simplex virus vector has all the characteristics (a) to (c).
[10] The pharmaceutical composition according to 8 or 9, wherein the region encoding the exogenous polypeptide is inserted into a deleted site of the ICP6 gene.
[11] The pharmaceutical composition according to any one of 8 to 10, which is for producing the exogenous polypeptide and inducing an immune response thereto.
[12] The pharmaceutical composition according to any one of 8 to 11, which is an agent for subcutaneous or intramuscular injection.
[13] The pharmaceutical composition according to any one of 8 to 12, wherein the exogenous polypeptide contains all or a part of a pathogen-derived protein.
[14] The pharmaceutical composition according to any one of 8 to 13, wherein the exogenous polypeptide contains all or a part of a spike protein derived from SARS-CoV-2.
   The present invention provides the followings.
[15] A method for producing an exogenous polypeptide and inducing an immune response thereto, the method comprising the step of administering a composition comprising an effective amount of a herpes simplex virus vector containing a region encoding the exogenous polypeptide and having two or more characteristics selected from the following characteristics (a) to (c) to a subject in need thereof. A herpes simplex virus vector or a composition comprising it for use in a method for producing an exogenous polypeptide and inducing an immune response thereto, the herpes simplex virus vector containing a region encoding the exogenous polypeptide and having two or more characteristics selected from the following characteristics (a) to (c). Use of a herpes simplex virus vector containing a region encoding an exogenous polypeptide and having two or more characteristics selected from the following characteristics (a) to (c) in manufacturing a composition for producing the exogenous polypeptide and inducing an immune response thereto.
   (a) the ICP6 gene is deleted or inactivated,
   (b) the γ34.5 gene is deleted or inactivated, and
   (c) the α47 gene is deleted or inactivated.
[16] The method, vector or composition comprising it, or use according to 15, wherein the herpes simplex virus vector has all the characteristics (a) to (c).
[17] The method, vector or composition comprising it, or use according to 15 or 16, wherein the region encoding the exogenous polypeptide is inserted into a deleted site of the ICP6 gene.
[18] The method, vector or composition comprising it, or use according to any one of 15 to 17, which is for producing the exogenous polypeptide in noncancerous cells.
[19] The method, vector or composition comprising it, or use according to any one of 15 to 18, wherein the composition is an agent for subcutaneous or intramuscular injection.
[20] The method, vector or composition comprising it, or use according to any one of 15 to 19, wherein the exogenous polypeptide contains all or a part of a pathogen-derived protein.
[21] The method, vector or composition comprising it, or use according to any one of 15 to 20, wherein the exogenous polypeptide contains all or part of a spike protein derived from SARS-CoV-2.
   The present invention also provides the followings.
[22] A method for producing an exogenous polypeptide in noncancerous cells, the method comprising the step of administering a composition comprising an effective amount of a herpes simplex virus vector containing a region encoding the exogenous polypeptide and having two or more characteristics selected from the following characteristics (a) to (c) to a subject in need thereof. A herpes simplex virus vector or a composition comprising it for use in a method for producing an exogenous polypeptide in noncancerous cells, the herpes simplex virus vector containing a region encoding the exogenous polypeptide and having two or more characteristics selected from the following characteristics (a) to (c). Use of a herpes simplex virus vector containing a region encoding an exogenous polypeptide and having two or more characteristics selected from the following characteristics (a) to (c) in manufacturing a composition for producing the exogenous polypeptide in noncancerous cells.
   (a) the ICP6 gene is deleted or inactivated,
   (b) the γ34.5 gene is deleted or inactivated, and
   (c) the α47 gene is deleted or inactivated.
[23] The method, vector or composition comprising it, or use according to 22, wherein the herpes simplex virus vector has all the characteristics of (a) to (c).
[24] The method, vector or composition comprising it, or use according to 22 or 23, wherein the region encoding the exogenous polypeptide is inserted into a deleted site at of the ICP6 gene.
[25] The method, vector or composition comprising it, or use according to any one of 22 to 24, which is for producing the exogenous polypeptide and inducing an immune response thereto.
[26] The method, vector, or composition comprising it, or use according to any one of 22 to 25, wherein the composition is an agent for subcutaneous or intramuscular injection.
[27] The method, vector or composition comprising it, or use according to any one of 22 to 26, wherein the exogenous polypeptide contains all or a part of a pathogen-derived protein.
[28] The method, vector or composition comprising it, or use according to any one of 22 to 27, wherein the exogenous polypeptide contains all or a part of a spike protein derived from SARS-CoV-2.

### Effects of the Invention

A cDNA of a relatively large size that encodes a full-length spike protein or the like can be incorporated into the virus vector of the present invention.

If the virus vector of the present invention is used as a vaccine, an immune response to eliminate the virus may promote vaccine effect.

If the virus vector of the present invention is used as a vaccine, a virus-derived protein may act as an adjuvant.

The vaccine effect of the virus vector vaccine provided by the present invention may not be attenuated by the presence of anti-herpes virus antibodies in blood, and therefore repeated administration of the virus vector vaccine may be effective.

The virus vector of the present invention uses herpes simplex virus (HSV) as the backbone, for which virus techniques for easily and accurately inserting an arbitrary cDNA have been established, and therefore vaccines against new viruses can be produced relatively quickly.

Methods for producing and purifying virus vector vaccines in large quantities at high titers have been established, and therefore the virus vector vaccine provided by the present invention is suitable for clinical applications.

### Brief Description of the Drawings

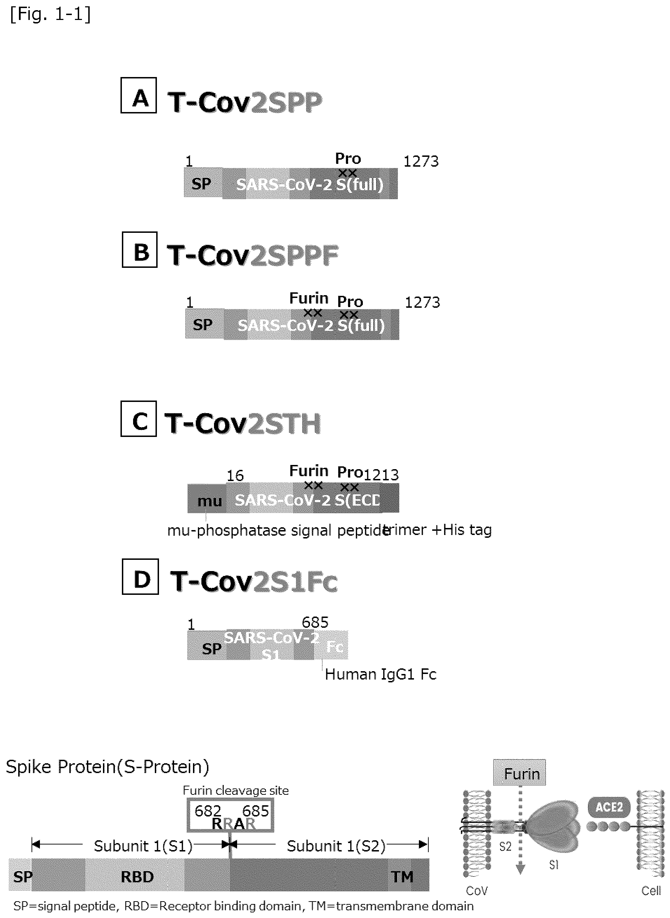

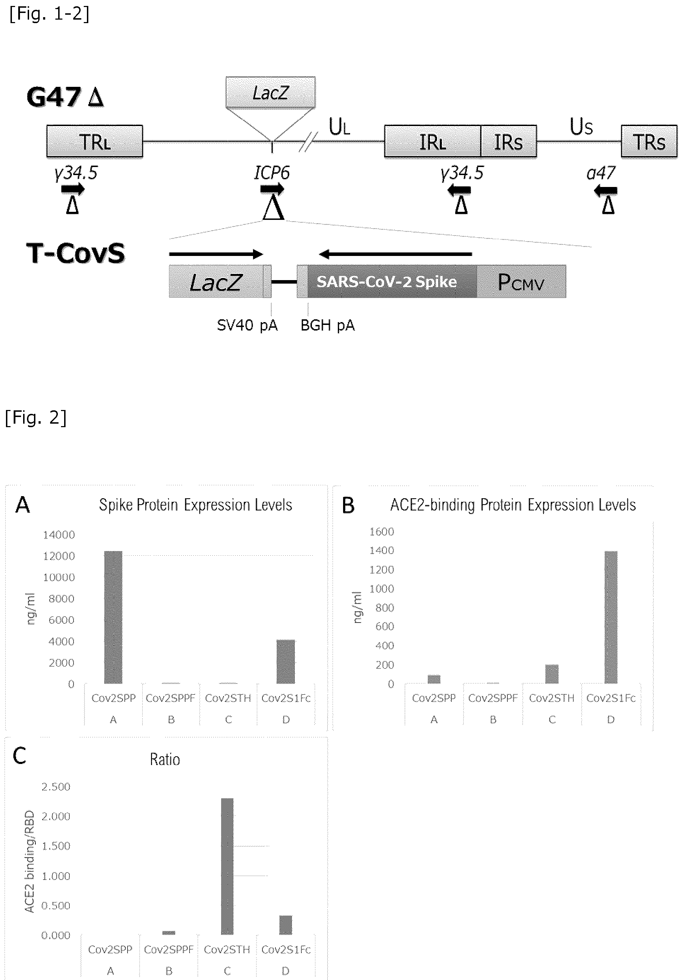

[Fig. 1-1] Structures of the polypeptides expressed from the constructs inserted into the virus vector used in the experiments.
[Fig. 1-2] Structure of the virus used in the experiment.
[Fig. 2] Expression of polypeptides in HEK293T cells transfected with expression plasmids of the respective constructs. A, expression levels of spike protein; B, expression levels of ACE2-binding protein; and C, ratio of expression levels of ACE2-binding protein to that of spike protein.
[Fig. 3] Expression of polypeptides in virus-infected Vero cells. A, expression levels of spike protein; B, expression levels of ACE2-binding protein; and C, ratio of expression levels of ACE2-binding protein to that of spike protein.
[Fig. 4] Expression levels of anti-Cov2 spike protein antibody.
[Fig. 5] Antibody concentrations in blood (OD450). A, (T-Cov2SSP), 1/10,000 and 1/1,000,000 dilution; B (T-Cov2SSPF), 1/10,000 and 1/1,000,000 dilution; C (T-Cov2STH), 1/100 dilution; D (T-Cov2S1Fc), 1/100 dilution; mock, 1/100 dilution; and T-01, 1/100 dilution.
[Fig. 6] Remaining levels of the virus in muscles after 8 weeks.
[Fig. 7] Pathological images after 8 weeks. HE staining and immunostaining using anti-HSV-1 antibody (green), anti-CD8 antibody (brown), and anti-CD4 antibody (red) was performed. The results are shown for the representative dose, 1 x 10⁷ pfu.
[Fig. 8] Weight changes of vaccine-administered mice.
[Fig. 9] Changes in expression levels of anti-Cov2 spike protein antibody.
[Fig. 10-1] Changes in expression levels of Cov2-spike protein. Left, intramuscular spike protein concentration; and right, blood spike protein concentration. The spike protein was detected in blood of only one animal out of n=3.
[Fig. 10-2] Time course for viral DNA levels in muscle
[Fig. 11] Time course for viral DNA levels in muscle. No viral DNA was detected in blood for all samples.
[Fig. 12-1] Images of muscles to which the respective viruses were administered (administration of 1 x 10⁷ pfu, day 1). HE staining and IHC using anti-HSV-1 antibody (green), anti-CD8 antibody (brown) and anti-CD4 antibody (red) were performed. HSV-1 positivity was observed along the administration site for both T-01 and A, T-Cov2SPP. Similar results were also obtained at a dose of 1 x 10⁶ pfu.
[Fig. 12-2] Images of muscles to which the respective viruses were administered (day 5, administration of 1 x 10⁷ pfu). HE staining and IHC using anti-HSV-1 antibody (green), anti-CD8 antibody (brown) and anti-CD4 antibody (red) were performed. Many CD8- and CD4-positive cells were observed, which might indicate inflammation at the administration site. Almost no HSV-1-positive cells were observed.
[Fig. 12-3] Images of muscles to which the respective viruses were administered (day 5, administration of 1 x 10⁶ pfu). HE staining and IHC using anti-HSV-1 antibody (green), anti-CD8 antibody (brown) and anti-CD4 antibody (red) were performed. Many CD8- and CD4-positive cells were observed. Almost no HSV-1-positive cells were observed.
[Fig. 13-1] Time course for viral DNA levels in muscle. The levels of virus in muscle at the administration site were almost the same from 1 to 4 weeks with only trace levels of copies detected.
[Fig. 13-2] Expression levels of anti-Cov2 spike protein antibody in blood. In the case of intramuscular (IM) administration, an increase in blood antibody concentration was observed from 1 to 3 weeks, followed by a decreasing tendency after 4 weeks. In the case of intratumoral (IT) administration, the blood antibody concentration was as high as about 35 times of that observed with the intramuscular administration in the same 3 weeks, despite that the administration dose was 1/5.

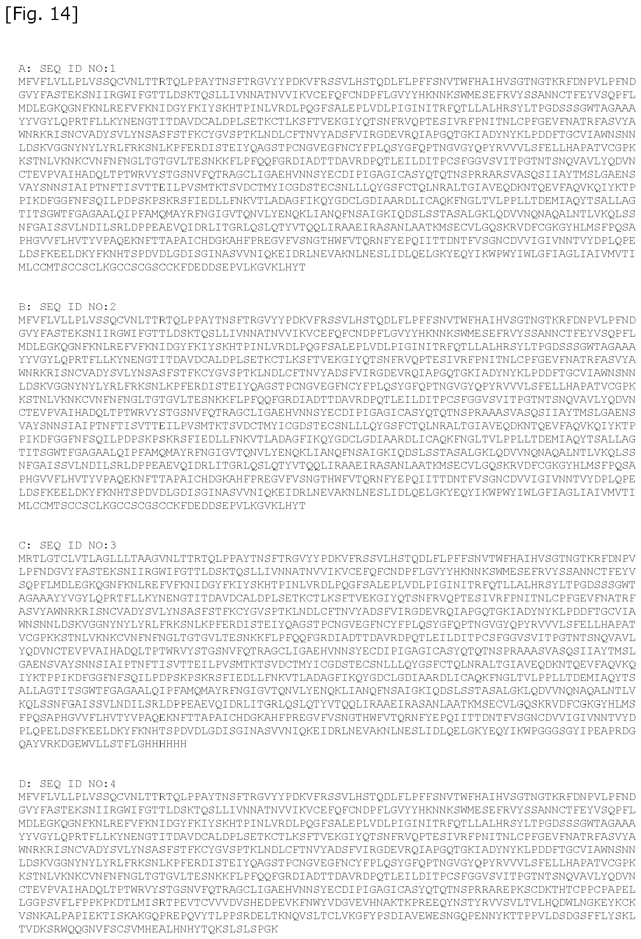

[Fig. 14] Amino acid sequences of the polypeptides used in the experiments.
[Fig. 15] Nucleotide sequence of LacZ-fused T-Cov2 spike(A) gene insertion site including upstream and downstream thereof.
[Fig. 16] Nucleotide sequence of the γ34.5 gene including upstream and downstream thereof (within the TR_{L} region).
[Fig. 17] Nucleotide sequence of the α47 gene including upstream and downstream thereof.

### Modes for Carrying out the Invention

Hereafter, the present invention will be explained in detail with reference to embodiments of the present invention (also referred to as the present embodiments). The following embodiments are exemplified to explain the present invention, and it is not intended to limit the present invention to only these embodiments.

### [Herpes simplex virus.]

In the present embodiments, a herpes simplex virus (HSV) containing a region encoding an exogenous polypeptide is used. When a virus is used as a tool to deliver a genetic material to nucleic acids, it may be referred to as virus vector. A genetically engineered virus may be referred to as mutant or recombinant virus.

The herpes simplex virus is classified in the genus *Simplexvirus* of the subfamily *Alphaherpesviridae* of the family *Herpesviridae.* Two types of the virus, type 1 (HSV-1) and type 2 (HSV-2), have been isolated so far. HSV-1 is preferably used in the present embodiments.

HSV-1 has an envelope and the mature particle has a size of 100 to 150 nm. It has a tegument inside the envelope and a capsid further inside the tegument, and the viral DNA is present in the capsid. The genome is double-stranded DNA. HSV-1 is also known to have the following characteristics.
1) It is infectious to all types of cells in human.
2) The life cycle and the genome sequence of the virus have been elucidated.
3) The functions of the majority of the viral genes have become clear and genetic manipulation can be applied.
4) The viral genome is large (about 152 kb), and therefore a large gene or multiple genes can be incorporated.
   HSV-1 further has the following advantages suitable for clinical application.
5) There are antiviral drugs to suppress proliferation thereof.
6) Anti-HSV-1 antibodies in blood may not attenuate the vaccine effect, and therefore repeated administration thereof is possible.
7) Preclinical evaluation of safety and effects can be performed in animals due to the existence of mice and monkeys that are sensitive to HSV-1.
8) The viral DNA is not incorporated into the genome of the host cell and is present outside the chromosome.
9) Methods for mass production and purification thereof have been established.

The genome of HSV-1 consists of two unique sequence regions: 82% of long unique region (U_{L}) and 12% of short unique region (Us), as well as terminal repeat (TR) and inverted repeat (IR) locating at each end of U_{L} and Us (Table 1, Todo, T.

(2008). Frontiers in Bioscience a Journal and Virtual Library 13, 2060-2064). Since each of the two regions L and S can take two directions independently, the HSV-1 genomic DNA has four isomers. This genome contains, in total, 84 genes encoded unidirectionally in R_{L}1 and R_{L}2 on TR_{L}, U_{L}1 to U_{L}56 on U_{L}, R_{S}1 on TR_{S}, and U_{S}1 to Us12 on Us; about half of which are genes unnecessary for viral growth, and deletion of these non-essential gene moieties can reduce pathogenicity and allow gene transduction (Carson, J. et al. (2010). Drugs of the Future 35, 183-195).

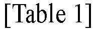

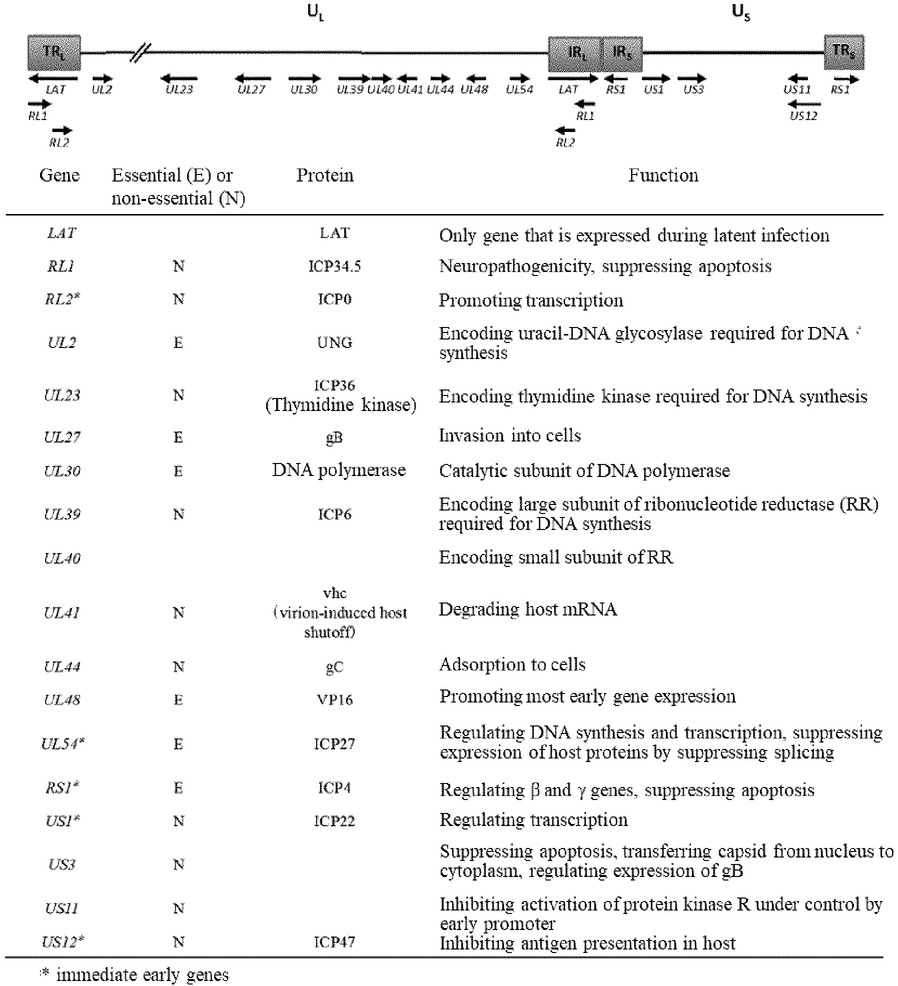

If the virus used in the present embodiments is an HSV-1 mutant, it can have one or more of the following characteristics.
- Deletion of virus replication ability in noncancerous cells due to inactivation of enzymes involved in viral DNA synthesis, such as thymidine kinase (TK), ribonucleotide reductase (RR), and uracil-N-glycosylase (UNG or UDG).
- Deletion of virus replication ability in noncancerous cells due to deletion of the gene γ34.5, which encodes the protein ICP34.5 involved in the pathogenicity of HSV-1.
- Promoted vaccine effect due to deletion of α47.
- Deletion or inactivation of genes for preventing reversion to wild type and thereby increasing safety (e.g., deletion or inactivation of the endogenous γ34.5 gene, α47 gene, α0 gene (ICP0 gene), U_{L}41 gene (vhs gene), and U_{L}56 gene).
- Enhancement of immunity and prolongation of survival by expression of immunestimulating genes (genes for IL-4, IL-10, GM-CSF, IL-12, soluble B7.1, etc.).

In one embodiment, the virus to be used is preferably an HSV-1 mutant having, among the following characteristics (a) to (c), at least one, more preferably two or more, further preferably all, of the characteristics:
(a) the ICP6 gene is deleted or inactivated,
(b) the γ34.5 gene is deleted or inactivated, and
(c) the α47 gene is deleted or inactivated.

With respect to the characteristics (a) and (b) mentioned above, deletion of ICP6, the large subunit of RR, which is the key enzyme for nucleotide metabolism and viral DNA synthesis in non-dividing cells, and/or γ34.5, which antagonizes the function of phosphorylated PKR that occurs during viral infection, results in a virus that cannot multiply in normal cells. With respect to the characteristic (c) mentioned above, the protein encoded by the α47 gene has an ability to escape from immunosurveillance of the host by inhibiting TAP to suppress expression of MHC class I on the host cell surface, and therefore α47 gene-deleted HSV-1 is expected to give a stronger stimulation to immune cells due to maintenance of the MHC class I expression of the host cells. Further, in HSV-1 having both of the above characteristics (b) and (c), the US11 late promoter, which overlaps with the α47 gene on the genome, is simultaneously deleted with the deletion of α47, so that the expression of the US11 gene is placed under the control of the α47 immediate-early promoter, resulting in earlier expression of the gene, and therefore it comes to have an ability to restore the viral replication ability, which has been weakened by the deletion of γ34.5, only in tumor cells.

For the present invention and embodiments thereof, the term deletion or inactivation of a gene means deletion of all or a part of the gene, substitution or modification of some nucleotides of the gene, insertion of an unnecessary sequence into the gene, or the like to suppress expression of the gene. Deletion or inactivation of a gene can be performed by known methods.

Examples of HSV of which γ34.5 gene, ICP6 gene and/or α47 gene are deleted or inactivated as described the characteristics (a) to (c) mentioned above include G207 in which two copies of the γ34.5 gene are both deleted and the ICP6 gene is inactivated, and G47Δ in which two copies of the γ34.5 gene are deleted, the ICP6 gene is inactivated, and the α47 gene is deleted. Therefore, the virus used for the present embodiments can also be produced by further modifying G207 or G47Δ.

In a particularly preferred embodiment, the virus to be used uses G47Δ as a basic skeleton. G47Δ is known as a replication-restricted recombinant HSV expressing the *E. coli* lacZ gene and subjected to deletion or inactivation of the γ34.5 gene, ICP6 gene (U_{L}39 gene), and α47 gene (ICP47 gene). Because of the wide therapeutic range thereof, it can be safely intracerebrally administered to humans at high doses, and was approved in Japan in June 2021 as a product for regenerative medicine etc. of which indication is malignant glioma (generic name, teserpaturev; product name, Delytact Injection). G47Δ was prepared from G207 derived from a virus wild strain (HSV-1 F strain) (Todo, T. et al. (2001). Proc Natl Acad Sci USA 98: 6396-6401).

With respect to the characteristic (a) mentioned above, it is preferred that the region encoding an exogenous polypeptide described below is inserted at the site where the ICP6 gene has been deleted or inactivated. For the present invention and embodiments thereof, the term region encoding a polypeptide means that the nucleotide sequence of the region encodes the amino acid sequence of the polypeptide as well as that a complementary sequence of the nucleotide sequence of the region encodes the amino acid sequence of the polypeptide.

The expression that a virus has the characteristic (a) described above and a region encoding an exogenous polypeptide is inserted at the site where the ICP6 gene has been deleted or inactivated means that, for example, the virus has a sequence that is highly identical to the portion of the sequence of SEQ ID NO: 5 mentioned in Sequence Listing other than Location 5349..9170, and a sequence encoding an immunogenic polypeptide or a complementary sequence thereof is inserted in place of the sequence of Location 5349..9170.

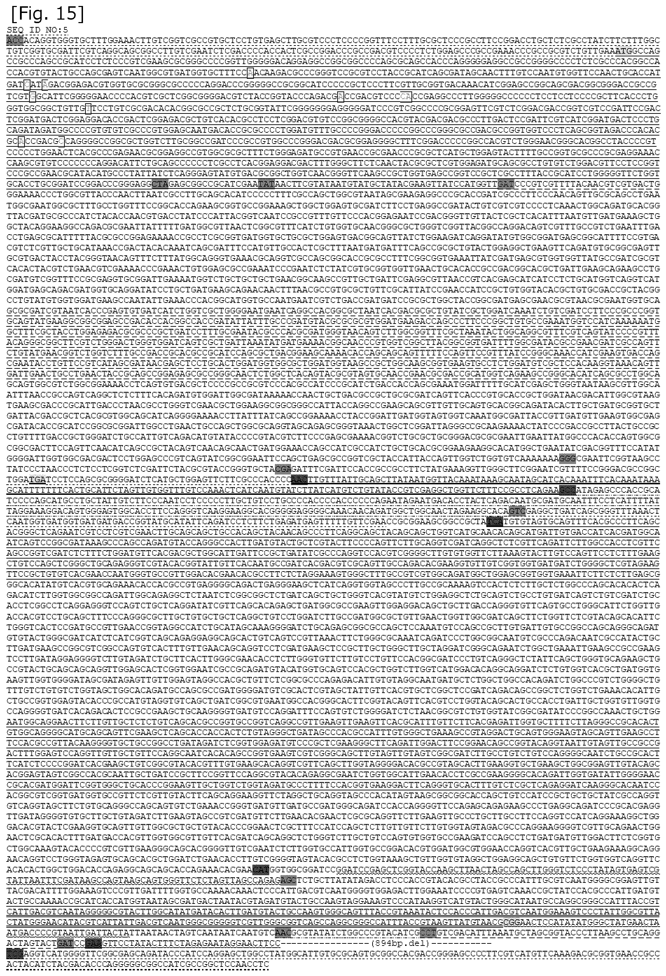

As SEQ ID NO: 5 and in Fig. 15, a nucleotide sequence of the LacZ-fused T-Cov2 spike(A) gene-inserted site including upstream and downstream sequences thereof is shown. The region encoding the exogenous polypeptide, i.e., the insertion site, corresponds to Location 5349..9170 in the sequence of SEQ ID NO: 5, or the sequence from the double-underlined TCA to the double-underlined CAT in Fig. 15 (the double-underlined TCA is the complementary sequence of the termination codon and the double-underlined CAT is the complementary sequence of the start codon).

The expression that the virus has the characteristic (b) mentioned above means that, for example, the virus has a sequence derived from the sequence of SEQ ID NO: 6 mentioned in Sequence Listing by deletion of all or a part of the sequence of Location 88...834, substitution or modification of some nucleotides thereof, or insertion of an unnecessary sequence into the same, which results in suppression of the expression of the γ34.5 gene, or that the virus has a sequence derived from the sequence of SEQ ID NO: 6 by deletion of all or a part of the nucleotide sequence in IR_{L} corresponding to Location 88..834 thereof, substitution or modification of some nucleotides thereof, or insertion of an unnecessary sequence into the same, which results in suppression of the expression of the γ34.5 gene. Ii is preferred that for the both γ34.5 gene sequences, all or parts of them are deleted, some nucleotides thereof are substituted or modified in them, or an unnecessary sequence is inserted into them, resulting in suppression of the expression of both genes. The expression that the virus has the characteristics (b) mentioned above also means that, for example, the virus has a sequence that is highly identical to the sequence of SEQ ID NO: 6 mentioned in Sequence Listing except for the sequence of Location 177..1128.

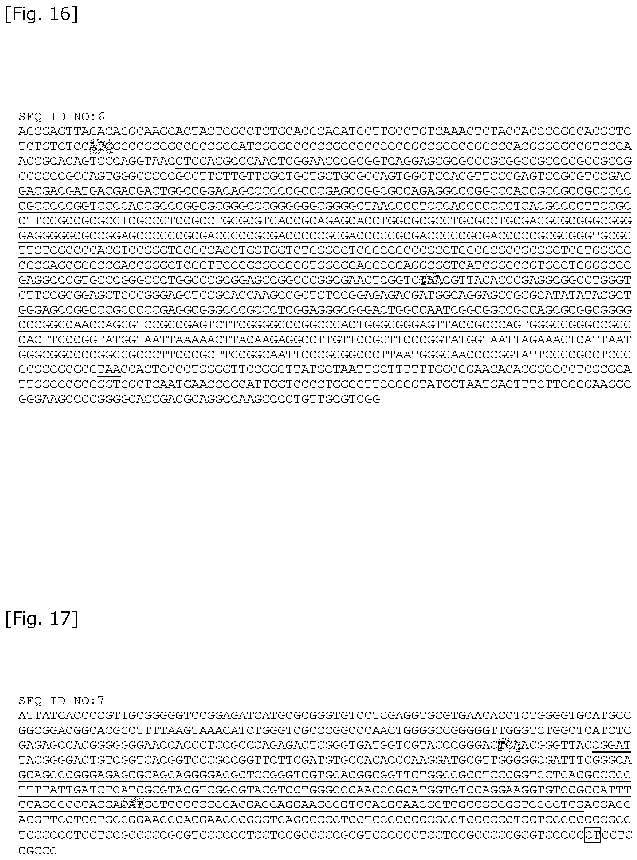

As SEQ ID NO: 6 and in Fig. 16, a nucleotide sequence of the γ34.5 gene of a strain from which G47Δ is derived including upstream and downstream nucleotide sequences thereof (within the TR_{L} region) is shown. The sequence of Location 88..834 in the sequence of SEQ ID NO: 6, and the sequence from the start codon to the termination codon shown with shading in Fig. 16 are the coding region of the γ34.5 gene. The site which is lacked in the basic skeleton of G47Δ (nucleotide numbers 576 to 1257 in GU734771.1) corresponds to Location 177..1128 in the sequence of SEQ ID NO: 6 or the underlined part in Fig. 16. The aforementioned sequence of TR_{L} and the complementary sequence around the coding region of the γ34.5 gene of IR_{L} (nucleotide numbers 124349 to 125798 in GU734771.1) are completely identical.

The expression that the virus has the characteristic (c) mentioned above means that, for example, the virus has a sequence derived from the sequence of SEQ ID NO: 7 mentioned in Sequence Listing by deletion of all or a part of the sequence of Location 218...484, substitution or modification of some nucleotides thereof, or insertion of an unnecessary sequence into the same, which results in suppression of the expression of the α47 gene. The expression that the virus has the characteristic (c) mentioned above also means that, for example, the virus has a sequence that is highly identical to the sequence of SEQ ID NO: 7 mentioned in Sequence Listing except for the sequence of Location 230...540.

As SEQ ID NO: 7 and in Fig. 17, a nucleotide sequence of the α47 gene of a strain from which G47Δ is derived, including upstream and downstream sequences thereof, is shown. The α47 gene is encoded on the complementary strand side. The sequence of Location 218..484 in the sequence of SEQ ID NO: 7, and the sequence from the start codon to the termination codon indicated with shading in Fig. 17 are the coding region of the α47 gene. The sequence of the site deleted in the basic skeleton of G47Δ (nucleotide numbers 576 to 1527 of GU734771.1) corresponds to the sequence of Location 177..1128 in the sequence of SEQ ID NO: 7 and the underlined portion in Fig. 17.

Whether a virus used in a certain pharmaceutical composition is a herpes simplex virus referred to in the present invention or embodiments thereof can be confirmed by methods well known to those skilled in the art such as gene amplification (PCR), enzyme immunoassay, highly sensitive fluorescent antibody (FA) method, Southern blot hybridization, liquid phase (nucleic acid) hybridization, and in situ hybridization.

### [Exogenous polypeptide]

In the present embodiments, the herpes simplex virus vector contains a region encoding an exogenous polypeptide. The term exogenous means that the peptide is derived from a source other than the herpes simplex virus. The polypeptide encoded by the region of the herpes simplex virus vector according to the present embodiments is not limited in size or type, as long as the polypeptide is produced by expression of the region and an immune response to it is induced in a subject to whom the virus is administered. The expression that an immune response is induced means that at least one of antibody production and cellular immunity is induced in the subject administered with the herpes simplex virus vector, unless especially stated. The property of the polypeptide to induce antibody production or cellular immunity may be referred to as immunogenicity.

The polypeptide used in the present embodiments is all or a part of a protein derived from a pathogen. The pathogen may be a virus, bacterium, fungus, rickettsia, parasite, or prion. The protein derived from a pathogen may be a polypeptide constituting the pathogen itself, such as a virus or bacterium, as well as a polypeptide of a toxin protein produced by the pathogen.

Examples of the pathogen include novel coronavirus (SARS-CoV-2), influenza A virus, influenza B virus, respiratory syncytial virus, parainfluenza virus, *Streptococcus pneumoniae, Corynebacterium diphtheriae, Clostridium tetani,* measles virus, mumps virus, rubella virus, rabies virus, *Staphylococcus aureus, Clostridium difficile,* human tuberculosis bacillus, *Candida albicans, Haemophilus influenzae B* (HiB), poliovirus, hepatitis B virus, human papilloma virus (L1, L2, E6, and E7), human immunodeficiency virus, *Helicobacter pylori, Staphylococcus aureus,* pertussis toxin, polio virus, *Staphylococcus aureus, Bacillus pertussis* (toxin), polio virus (VP1 to VP4), and *Plasmodium falciparum malaria.*

In one embodiment, the polypeptide is derived from a cancer-causing pathogen. It is known that infections with certain pathogens cause cancer. For example, hepatitis B and C viruses cause liver cancer; human papilloma virus (HPV) causes cervical, penile, vulvar, vaginal, anal, oral, and pharyngeal cancers; *Helicobacter pylori* causes stomach cancer; Epstein-Barr virus (EBV) causes Burkitt lymphoma, Hodgkin's lymphoma, and pharyngeal cancer; human T-cell leukemia virus type I (HTLV-1) causes adult T-cell leukemia and lymphoma; and so forth.

In one embodiment, the polypeptide can be a cancer antigen. Cancer antigen is a protein that is expressed at a higher level in cancer cells than in normal cells or is specifically expressed in cancer cells, etc. It is being investigated to treat cancer or inhibit cancer recurrence by using a cancer-derived peptide epitope, portion of individualized cancer antigen or portion of a cancer hotspot antigen (e.g., WO2020/097291).

The exogenous polypeptide used in the present embodiments may be relatively large in size. For example, it may be 500 amino acid residues long, 1000 amino acid residues long, or 1500 amino acid residues long. Because of the large genome size of herpes simplex virus, it is possible to incorporate a region encoding a polypeptide of large size or multiple polypeptides.

The location of introduction of the region encoding the exogenous polypeptide is not particularly limited. For example, the region encoding the exogenous polypeptide can be introduced at a location where a non-essential gene of the virus has been deleted or inactivated. When the virus is HSV-1, the non-essential genes shown in Table 1 mentioned above can be deleted and the region encoding the exogenous polypeptide can be introduced. In a particularly preferred embodiment, the region encoding the exogenous polypeptide is inserted at the site where the ICP6 gene has been deleted or inactivated, as described above. The expression of the introduced region can be confirmed by using known methods, as shown in the examples mentioned below.

For insertion of the region encoding the exogenous polypeptide into herpes simplex virus, for example, the G47Δ-BAC system, T-BAC system, or similar systems described in Non-patent document 1 to 5, Patent documents 1 to 4 mentioned above, and WO2005/103237 can be used. The T-BAC system is a modification of the G47Δ-BAC system (Non-patent document 4 mentioned above), which allows production of HSV-1 with a replication ability higher than that of G47Δ-BAC product and equivalent to that of G47Δ.

The T-BAC system is a technique for the production of recombinant HSV-1 that facilitates the maintenance and amplification of the G47Δ genome by inserting the bacterial artificial chromosome (BAC), a cloning vector based on a replicon of the F factor plasmid that can stably retain a large DNA fragment up to 1 million bp in a single copy in *E. coli,* into the ICP6 deletion site of the G47Δ genome. This T-BAC contains the loxP and FRT sequences, and by mixing it with a shuttle vector plasmid that also contains loxP and FRT sequences, and utilizing two recombinase systems, Cre/loxP and FLP/FRT, insertion of an exogenous gene carried by the shuttle vector and excision of BAC can be caused, allowing the production of a recombinant HSV-1 having the exogenous gene inserted into the G47Δ basic skeleton.

In one embodiment, the region encoding the exogenous polypeptide is introduced into a herpes simplex virus so that the polypeptide is produced in noncancerous cells. Noncancerous cells mean cells that are not cancer cells. Those skilled in the art can appropriately determine whether cells are noncancerous or not. Whether production of a target polypeptide in noncancerous cells and an immune response thereto are induced or not can be evaluated by using HSV-1-susceptible experimental animals such as A/J mice, DBA/2 mice, and BALB/c mice.

### [Use as novel coronavirus vaccine]

In one embodiment, the exogenous polypeptide is derived from a novel coronavirus (SARS-CoV-2). More specifically, such an exogenous polypeptide may contain the wild-type leader sequence derived from SARS-CoV-2, the full length spike protein, and double-proline (PP) mutation for conformational stabilization of the spike protein. Such a polypeptide is, for example, any one of the following polypeptides (i) to (iii):
(i) the polypeptide consisting of the amino acid sequence of SEQ ID NO: 1;
(ii) a polypeptide consisting of an amino acid sequence derived from the amino acid sequence of SEQ ID NO: 1 by substitution, deletion, insertion, and/or addition of one or more amino acids, wherein the amino acids corresponding to the positions 986 and 987 of the sequence of SEQ ID NO: 1 are P and P in that order, and which can induce an immune response; and
(iii) a polypeptide consisting of an amino acid sequence having an identity of at least 80% to the amino acid sequence of SEQ ID NO: 1, wherein the amino acids corresponding to the positions 986 and 987 of the sequence of SEQ ID NO: 1 are P and P in that order, and which can induce an immune response.
   In another preferred embodiment, the exogenous polypeptide may contain the wild-type leader sequence derived from SARS-CoV-2, full length spike protein, and double-proline (PP) mutation for conformational stabilization of the spike protein, as well as deletion of a site consisting of the amino acid sequence RRAR (SEQ ID NO: 8) recognized and cleaved by furin, a protease of the host cell (furin cleavage site). Such a polypeptide is, for example, any one of the following polypeptides (iv) to (vi):
(iv) the polypeptide consisting of the amino acid sequence of SEQ ID NO: 2;
(v) a polypeptide consisting of an amino acid sequence derived from the amino acid sequence of SEQ ID NO: 2 by substitution, deletion, insertion, and/or addition of one or more amino acids, wherein the amino acids corresponding to the positions 683, 685, 986, and 987 of the sequence of SEQ ID NO: 2 are A, A, P and P in that order, and which can induce an immune response; and
(vi) a polypeptide consisting of an amino acid sequence having an identity of at least 80% to the amino acid sequence of SEQ ID NO: 2, wherein the amino acids corresponding to the positions 683, 685, 986, and 987 of the sequence of SEQ ID NO: 2 are A, A, P and P in that order, and which can induce an immune response.

For the present invention and embodiments thereof, deletion of an amino acid sequence that is recognized and cleaved by a certain enzyme means such deletion of all or a part of the amino acid sequence, substitution or modification of a part of the amino acids of the sequence, or insertion of an unnecessary sequence into the sequence that the amino acid sequence is no longer recognized and cleaved by the enzyme.

In another preferred embodiment, the exogenous polypeptide contains a leader sequence derived from SARS-CoV-2 and containing a mutation, the extracellular domain (ECD) alone of the spike protein, PP mutation, deletion of the furin cleavage site, and further contains trimerization domain at the C-terminus. Such a polypeptides is, for example, any one of the following polypeptides (vii) to (ix):
(vii) the polypeptide consisting of the amino acid sequence of SEQ ID NO: 3;
(viii) a polypeptide consisting of an amino acid sequence derived from the amino acid sequence of SEQ ID NO: 3, by substitution, deletion, insertion, and/or addition of one or more amino acids in other than the regions consisting of the amino acids of the positions 1 to 20 and 1219 to 1256 (i.e., the part consisting of the amino acids of the positions 21 to 1218), which can induce an immune response; and
(ix) a polypeptide consisting of an amino acid sequence having an identity of at least 80% to the part of the amino acid sequence of SEQ ID NO: 3 other than the regions consisting of the amino acids of the positions 1 to 20 and 1219 to 1256 (i.e., the part consisting of the amino acids of the positions 21 to 1218), which can induce an immune response.

**In** another preferred embodiment, the exogenous polypeptide contains the wild-type leader sequence and subunit 1 (S1) of the spike protein derived from SARS-CoV-2, and contains the Fc region of human IgG at the C-terminus. Such a polypeptide is, for example, any one of the following polypeptides (x) to (xii):
(x) the polypeptide consisting of the amino acid sequence of SEQ ID NO: 4;
(xi) a polypeptide consisting of an amino acid sequence derived from the amino acid sequence of SEQ ID NO: 4 by substitution, deletion, insertion, and/or addition of one or more amino acids, which can induce an immune response; and
(xii) a polypeptide consisting of an amino acid sequence having an identity of at least 80% to the amino acid sequence of SEQ ID NO: 4, which can induce an immune response.

In the present embodiments, the amino acids may be natural amino acids as well as derivatives thereof, artificial amino acids, and non-natural amino acids. The amino acids used for the present embodiments are preferably, in one aspect, natural amino acids.

In the explanations of the present invention and embodiments thereof, for amino acids or amino acid residues, A stands for alanine, C for cysteine, D for aspartic acid, E for glutamic acid, F for phenylalanine, G for glycine, H for histidine, I for isoleucine, K for lysine, L for leucine, M for methionine, N for asparagine, P for proline, Q for glutamine, R for arginine, S for serine, T for threonine, U for selenocysteine, V for valine, W for tryptophan, and Y for tyrosine, unless especially noted.

### [Identity of nucleotide sequence or amino acid sequence]

For the present invention and embodiments thereof, the number of amino acids to be replaced or the like mentioned in the expression that "an amino acid sequence derived by substitution, deletion, insertion, and/or addition of one or more amino acids" is not particularly limited for any proteins as long as the protein consisting of the amino acid sequence has the desired function, unless especially noted. For example, the number may be about 1 to 250, 1 to 200, 1 to 150, 1 to 100, 1 to 50, 1 to 40, 1 to 30, 1 to 20, 1 to 15, 1 to 9, or 1 to 4, or if substitution etc. is made with amino acids having similar properties, substitution etc. for amino acids of a further larger number may be possible. Means for preparing polynucleotides or proteins concerning such amino acid sequences are well known to those skilled in the art.

The term "identity" used in the present invention and embodiments thereof for nucleotide sequence (also referred to as base sequence) or amino acid sequence means percentage of the number of matching nucleotides or amino acids shared between two sequences aligned in an optimal manner, unless especially stated. That is, it can be calculated in accordance with the following equation: Identity = (Number of matched positions/Total number of positions) x 100, and can be calculated by using commercially available algorithms. Such algorithms are incorporated into the NBLAST and XBLAST programs described in Altschul et al. J. Mol. Biol. 215(1990)403-410. In more detail, searches and analyses for nucleotide or amino acid sequence identities can be performed by using algorithms or programs well known to those skilled in the art (e.g., BLASTN, BLASTP, BLASTX, and ClustalW). When using such programs, the parameters can be set appropriately by those skilled in the art, or the default parameters of each program can be used. The specific methods of these analyses are also well known to those skilled in the art. The genetic information processing software GENETIX (registered trademark, Genetix, Inc.) may also be used to calculate the identity. If the sequence for which the % identity is sought has an additional sequence at the terminus, such as a tag sequence, that is not present in the sequence to be compared, the additional sequence portion is not included in the calculation of the identity %.

The term "high identity" used for nucleotide sequence or amino acid sequence in the present invention and embodiments thereof means, in any case, a sequence identity of at least 50%, such as 60% or higher or 70% or higher, preferably 80% or higher, more preferably 85% or higher, further preferably 90% or higher, further preferably 95% or higher, further preferably 97.5% or higher, further preferably 99% or higher.

The polypeptides, proteins, polynucleotides, DNAs, and genes used in the present invention and embodiments thereof can be produced by methods known to those skilled in the art.

### [Pharmaceutical composition]

The present embodiments also relate to a pharmaceutical composition comprising a virus that is used as the vector described above as one aspect of the present invention. The pharmaceutical composition may be used to produce the exogenous polypeptide in noncancerous cells, preferably to induce an immune response against the exogenous polypeptide in a subject to whom it has been administered, more preferably to produce antibodies against the exogenous polypeptide and induce cellular immunity in a subject to whom it has been administered.

If the exogenous polypeptide encoded in the contained region is derived from an infectious disease-causing pathogen, the pharmaceutical composition of the present embodiments can be used as a vaccine for treatment of an infectious disease caused by that pathogen, preferably for prevention of the infectious disease.

Examples of the infectious disease include viral infections (e.g., novel coronavirus infections, influenza, acquired immunodeficiency syndrome (AIDS), adult T-cell leukemia, Ebola hemorrhagic fever, viral hepatitis, viral meningitis, yellow fever, cold syndrome, rabies, cytomegalovirus infection, severe acute respiratory syndrome (SARS), progressive multifocal leukoencephalopathy, varicella, herpes zoster, hand-foot-and-mouth disease, dengue fever, erythema infectiosum, infectious mononucleosis, smallpox, rubella, acute poliomyelitis (polio), measles, pharyngoconjunctival fever (pool fever), Marburg hemorrhagic fever, hantavirus renal hemorrhagic fever, Lassa fever, mumps, West Nile fever, herpangina, and chikungunya fever), bacterial infections, rickettsial infections, parasitic infections, and prion diseases.

If the exogenous polypeptide encoded in the contained region is derived from a cancer-causing pathogen, the pharmaceutical composition of the present embodiments can be used as a vaccine for prevention of a specific cancer. Cancers to which the present invention is applicable include liver cancer, cervical cancer, penile cancer, vulvar cancer, vaginal cancer, anal cancer, oral cancer, pharyngeal cancer, gastric cancer, Burkitt's lymphoma, Hodgkin's lymphoma, and adult T-cell leukemia lymphoma.

When the exogenous polypeptide encoded in the contained region is derived from a cancer antigen, the pharmaceutical composition of the present embodiments can be used for treatment of a specific cancer, preferably as a vaccine for prevention thereof, vaccine for therapeutic treatment thereof, or vaccine for prevention of recurrence thereof. Examples of cancer for which application of the present invention is expected include, but are not limited to, brain tumor, glioma, ocular tumor, neuroblastoma, retinoblastoma, primary malignant lymphoma of CNS, meningioma, pituitary adenoma, schwannoma, craniopharyngioma, intraocular tumor, retinoblastoma, choroidal malignant melanoma, intraocular malignant lymphoma, tumor of ocular appendage, eyelid tumor, lacrimal gland cancer, ocular appendage lymphoma, orbital sarcoma, conjunctival tumor, optic nerve tumor, glioma, oral cavity cancer, tongue cancer, pharyngeal cancer, thyroid cancer, auricular cancer, adenoid cystic carcinoma, olfactory neuroblastoma, sarcoma of head and neck, lung cancer, breast cancer, thymoma, thymic carcinoma, malignant pleural mesothelioma, sarcoma of trunk, sarcoma of heart, pulmonary neuroendocrine tumor, SMARCA4-deficient thoracic tumor, non-small cell lung cancer, small cell lung cancer, esophageal cancer, stomach cancer, colorectal cancer (colon cancer and rectum cancer), small intestine cancer (duodenum cancer, jejunum cancer, and ileum cancer), GIST (gastrointestinal stromal tumor), pancreatic and gastrointestinal neuroendocrine tumors, anal cancer, gastrointestinal neuroendocrine tumor, hepatocellular cancer, biliary tract cancer (bile duct cancer (including intrahepatic bile duct cancer), gall bladder cancer, and papillary duodenal cancer), pancreatic cancer, pancreatic and gastrointestinal neuroendocrine tumors, liver tumor, pancreatic neuroendocrine tumor, renal cell carcinoma, renal pelvis and ureter cancers, bladder cancer, urachal carcinoma, renal tumor, prostate cancer, breast cancer, cervical cancer, uterine cancer (endometrial cancer), uterine sarcoma, ovarian and oviduct cancers, vaginal cancer, vulvar cancer, malignant melanoma (skin), basal cell carcinoma, spinous cell carcinoma, lymphoma of skin, soft tissue sarcoma (adults), desmoid tumor, osteosarcoma (pediatric), Ewing's sarcoma (pediatric), soft tissue sarcoma (pediatric), rhabdomyosarcoma (pediatric), undifferentiated/unclassifiable sarcomas, osteosarcoma, chondrosarcoma, chordoma, Ewing's sarcoma, liposarcoma, fibrosarcoma, myxofibrosarcoma, undifferentiated pleomorphic sarcoma, leiomyosarcoma, rhabdomyosarcoma, hemangiosarcoma, malignant peripheral nerve sheath tumor, synovial sarcoma, epithelioid sarcoma, alveolar soft part sarcoma, clear cell sarcoma, extraskeletal Ewing's sarcoma, acute myeloid leukemia, acute lymphocytic leukemia/lymphoblastic lymphoma, chronic myeloid leukemia, myelodysplastic syndrome, malignant lymphoma, B cell lymphoma, T/NK cell lymphoma, Hodgkin lymphoma, lymphoma of skin, chronic lymphocytic leukemia/small lymphocytic lymphoma, adult T-cell leukemia lymphoma, multiple myeloma, acute promyelocytic leukemia, follicular lymphoma, MALT lymphoma, lymphoplasmacytic lymphoma, mantle cell lymphoma, diffuse large B-cell lymphoma, peripheral T-cell lymphoma, Burkitt's lymphoma, extranodal NK/T-cell lymphoma, cancer of unknown primary origin, hereditary and familial tumors, neuroendocrine cancer, germ cell tumor, paraganglioma, germ cell tumor, testicular germ cell tumor, ovarian germ cell tumor, extragonadal germ cell tumor, and neuroendocrine tumor.

For the present invention and embodiments thereof, the term treatment referred to in relation to a disease or condition, it includes prevention, reduction of the risk of developing the disease, therapeutic treatment, inhibition of progression, and prevention of recurrence, unless especially noted.

The pharmaceutical composition of the present embodiments contains an effective amount of the virus as the active ingredient. For the present invention and embodiments thereof, the term effective amount means at least one of such an amount that an objective polypeptide is produced in noncancerous cells in a subject, such an amount that an immune response to the exogenous polypeptide is induced in the subject, and such an amount that an antibody to the exogenous polypeptide is produced in the subject, when it is administered to the subject. Specific doses can be appropriately determined by those skilled in the art according to the degree of symptoms, age, sex, weight, sensitivity difference of the target of the administration, administration method, time of administration, administration interval, nature of formulation, strength of promoter, and so forth.

The pharmaceutical composition of the present embodiments can be administered in an amount of, for example, about 10¹ to 10¹² plaque-forming units (pfu), preferably about 10⁷ to 10¹⁰ pfu, more preferably about 10⁸ to 5 x 10⁹ pfu, which can be administered by one or several times of injection. The number of times of the administration of the pharmaceutical composition of the present embodiments can be appropriately set according to the target patient and the target disease. For example, 2 to 5 weeks after the first administration, the second administration can be performed. If necessary, as the third and subsequent administrations, administration can be repeated necessary times every 1 to 8 months.

The administration method of the pharmaceutical composition the present embodiments is not particularly limited and can be administered, for example, intravenously, intraarterially, intracerebroventricularly, intraperitoneally, intrathoracically, into the spinal cavity, subcutaneously, intradermally, intraepidermally, intramuscularly, or on mucosal surfaces (for example, eye, nasal cavity, lung, oral cavity, bowel, rectum, vagina, and urinary tract surfaces). If the pharmaceutical composition is a vaccine for prevention of an infectious disease, it is preferably administered by subcutaneous or intramuscular injection. If the pharmaceutical composition is a vaccine for prevention of a cancer, it can be administered by subcutaneous or intramuscular injection. If the pharmaceutical composition is for therapeutic treatment of a cancer, it can be administered directly to the cancer (intratumoral administration). By intratumoral administration, a higher efficacy can be expected with a smaller dose.

The pharmaceutical composition of the present embodiments can be formulated by known formulation methods. For example, the pharmaceutical composition may contain an adjuvant or an arbitrary carrier, or it may simply be diluted in a physiologically acceptable solution such as sterile physiological saline or sterile buffered physiological saline without adding any adjuvant or carrier. It may also be made as a frozen, dried, or lyophilized formulation suitable for long-term storage.

The pharmaceutical composition of the present embodiments may contain one or more types of pharmaceutically acceptable additives. Examples of such additives include buffers, isotonic agents, preservatives, antioxidants, stabilizers, absorption enhancers, excipients, binders, lubricants, disintegrants, coloring agents, taste and odor correction agents, emulsifiers, surfactants, dissolution aids, suspending agents, and so forth.

When the pharmaceutical composition of the present invention is in the form of a subcutaneous or intramuscular injection, the pharmaceutical composition can be a formulation containing, for example, in addition to the active ingredient, L-histidine 6 mg, L-histidine hydrochloride hydrate 2 mg, sodium chloride 10 mg, magnesium chloride 1 mg, sodium edetate hydrate 0.2 mg, purified white sugar 375 mg, anhydrous ethanol 20 mg, and polysorbate 805 mg in one vial (5 mL).

The pharmaceutical composition of the present embodiments may further contain another active ingredient or may be used in combination with a pharmaceutical composition comprising another active ingredient. The pharmaceutical composition of the present invention can also be used in combination with another therapy.

Unless otherwise specified, all terms used herein (including technical and scientific terms) has the same meaning as widely understood by those skilled in the art to which the present invention belongs. The terms used herein are, unless otherwise specifically defined, to be construed as having consistent meanings with the meanings in the present description and in the related art, and are not to be interpreted in an idealized or excessively formatted sense.

### Examples

### [Preparation of 4 types of T-Cov2]

The structures of the polypeptides produced by the viruses used in the following experiments are shown in Fig. 1-1. A is one for preparing T-Cov2SPP virus, which contains the wild type leader sequence derived from SARS-CoV-2, the full length of the spike protein, and double proline (PP) mutation for conformationally stabilizing the spike protein. B is one for preparing T-Cov2SPP virus, which is derived from the structure of A by further deletion of the site consisting of the amino acid sequence RRAR (SEQ ID NO: 8), which is recognized and cleaved by furin, a protease of the host cell (furin cleavage site). C is one for preparing T-Cov2STH virus, which contains the mu-phosphatase leader sequence, the extracellular region alone of the spike protein (ECD), PP mutation, deletion of the furin cleavage site, and trimerization domain at the C-terminus. D is one for preparing Cov2S1Fc virus, which contains the wild-type leader sequence, subunit 1 (S1) of the spike protein, and the Fc region of human IgG at the C-terminus.

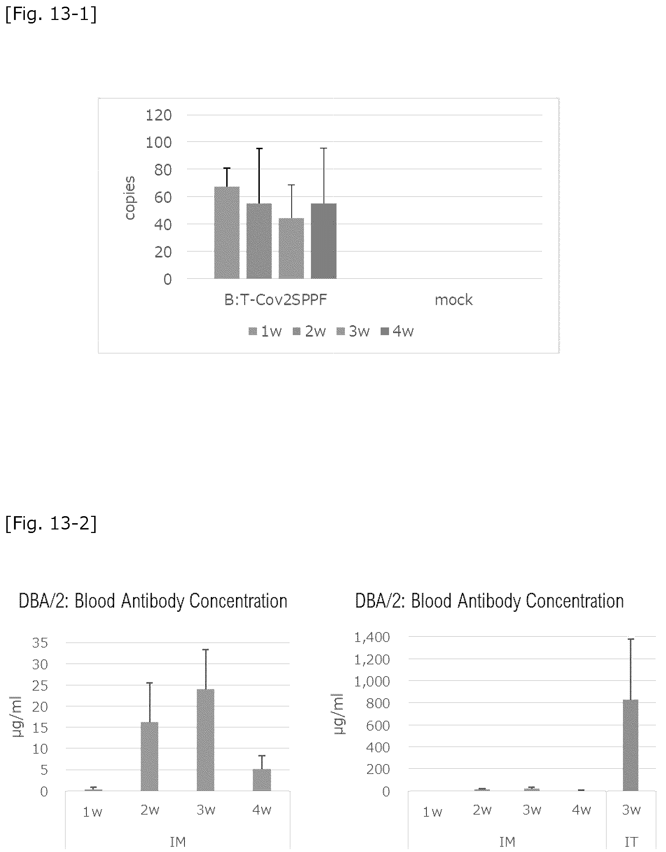

The amino acid sequences of the polypeptides A to D are shown as SEQ ID NOS: 1 to 4 in Sequence Listing and Fig. 13. Characteristics of the sequences are mentioned below.
A (SEQ ID NO: 1): Spike protein (GenBank: QHD43416.1), PP (K986P, V987P) mutation
B (SEQ ID NO: 2): Spike protein (GenBank: QHD43416.1), Furin (R683A R685A), PP (K986P, V987P) mutation
C (SEQ ID NO: 3): Spike protein (GenBank: QHD43416.1), Furin (R683A R685A), PP (K986P, V987P) mutation, mu-phosphatase signal peptide (1..20), C-terminal; GGGSG-YIPEAPRDGQAYVRKDGEWVLLLSTFL (fold on trimerization motif, 1219..1249) + G-HHHHHH (hexa-histidine tag, 1250..1256)
D (SEQ ID NO: 4): Spike S1 (1..685) (GenBank: QIC53204.1, Met1..Arg685), Fc region of human IgG1 (686..917) (GenBank: CAR58103.1, Glu98..Lys329)

The structure of the viruses used in the examples is shown in Fig. 1-2. The basic skeleton of the viruses was G47Δ having or subjected to deletions in two of γ34.5 present in TR_{L} and IR_{L}, inactivation by insertion of the LacZ gene into ICP6 present in U_{L}, and deletions in α47 present in Us and in the overlapping US11 promoter region. The T-BAC system described above was used to generate the viruses.

In detail, the viruses were created by inserting LacZ, polyA of SV40 (SV40 pA), inverted cytomegalovirus promoter (PCMV) and any one of the four types of the structures A to D (indicated as SARS-CoV-2 Spike in Fig. 1- 2), and polyA of BGH (BGH pA) in the downstream of them into the basic skeleton G47Δ at the ICP6 gene deletion site. Virus T-01 obtained by inserting only LacZ and PCMV, in which no exogenous gene was inserted at the ICP6 gene deletion site, was used as a control.

The nucleotide sequence of LacZ-fused T-Cov2 spike (A) gene insertion site used in the experiments, including the upstream and downstream sequences thereof, is shown as SEQ ID NO: 5 in Sequence Listing and Fig. 15. The sequence characteristics are shown below.

The dotted line (Location 1..226 in SEQ ID NO: 5) indicates the sequence upstream of the U_{L}39 gene coding region reported to contain the U_{L}39 gene promoter, the underline (Location 227..1544) indicates the amino acid coding region of the U_{L}39 gene (nucleotide numbers ~87656 in GU734771.1), the sequence indicated with the wavy line and the following sequence (from Location 1545) is an insertion sequence, the wavy line indicates the sequence resulting from the genetic recombination (Note 1: this sequence contains the loxP sequence), the broken line indicates the amino acid coding region of the lacZ gene, the following underline indicates the sequence derived from the plasmid vector (Note 2: sequence on the 3' side of lacZ derived from pcDNA6-E/Uni-lacZ), the dotted line also indicates a sequence derived the plasmid vector (Note 2: sequence of SV40 polyA and sequence around BGH polyA derived from pVP22/myc-His2), TGA is a termination codon of the ICP6-lacZ fusion protein, the double wavy line indicates the SV40 polyA sequence derived from pVP22/myc-His2, the two-dot chain line indicates the complementary sequence of BGH polyA derived from pVP22/myc-His2, the dotted line indicates sequences derived from the plasmid vector (sequence of SV40 polyA and sequence around BGH polyA derived from pVP22/myc-His2), the following underline indicates the complementary sequence of the region encoding the amino acid sequence of the T-Cov2 spike protein (A) (double-underlined TCA is the complementary sequence of the termination codon, and double-underlined CAT is the complementary sequence of the start codon) (TCA to CAT, Location 5349..9170), the double line indicates the complementary sequence of the CMV promoter, the wavy lines of the upstream and downstream thereof are sequences derived from the plasmid used (MCS), the two-dot chain line indicates a sequence adjacent to the CMV promoter sequence of pVP22/myc-His2, the broken line indicates a sequence generated at the time of the genetic recombination (synthetic oligomer sequence containing MCS incorporated in the production process of pLLZMF2 from pLLZF2), the wavy line indicates the complementary sequences of the adapter sequence and FRT sequence used in the genetic recombination, and the sequence indicated with the dotted line and following sequence (from nucleotide numbers 88551 in GU734771.1) are the sequences of ICP6 on the 3' side downstream of the deletion site of 894 bp (nucleotide numbers 87657 to 88550 in GU734771.1). *E. coli* β-galactosidase is expressed as a fusion protein with the N-terminus of ICP6. The parts (9 locations) as the differences of this sequence from GU734771.1 are indicated with boxes.

The nucleotide sequences of the γ34.5 gene of HSV-1 from which G47Δ is derived and upstream and downstream sequences thereof (within the TR_{L} region) are shown as SEQ ID NO: 6 in Sequence Listing and Fig. 16. In Fig. 16, the coding region of the γ34.5 gene is from the start codon to the termination codon indicated with shading (Location 88..834 in SEQ ID NO: 6). In Fig. 15, the site that is deleted in the generated virus (nucleotide numbers 576 to 1527 of GU734771.1) is underlined (Location 177..1128 in SEQ ID NO: 6). As for the sequence following the deletion site, the non-coding region on the 3' side of the γ34.5 gene to the termination codon (double-underlined) that occurs 130 bp downstream is translated as a nonsense amino acid sequence.

The above sequence of TR_{L} (around the coding region of the γ34.5 gene; nucleotide numbers 400 to 1849 of GU734 771.1) and the complementary sequence of the sequence around the coding region of the γ34.5 gene in IR_{L} (nucleotide numbers 124349 to 125798 of GU734771.1) are completely identical. The γ34.5 gene deletion site in IR_{L} corresponds to the nucleotide numbers 124671 to 125622 in GU734771.1 (complementary sequence). This sequence contains no part that differs from GU7347771.1.

The sequence of the α47 gene of HSV-1 from which G47Δ is derived, and the upstream and downstream sequences thereof are shown as SEQ ID NO: 7 in Sequence Listing and in Fig. 17. The sequence characteristics are described below.

The α47 gene is encoded on the complementary strand side. In Fig. 17, the sequence between the termination codon and the start codon indicated with shading is the coding region of the α47 gene (Location 218..484 in SEQ ID NO: 7). In Fig. 16, the region that was deleted in the generated virus is underlined (Location 230..540 in SEQ ID NO: 7). In the generated virus, the α47 gene was deleted from the translation start site and no new ORF was generated. The sequence of this part is derived from HSV-1 strain 17, as described in Assessment of Impact on Biodiversity of the "recombinant human herpes simplex virus type 1 (derived from F strain) (T-hIL12) expressing fused human IL-12 gene and *E. coli* lacZ gene and having inactivated γ34.5 gene, UL39 gene and α47 gene" approved on May 31, 2019 (https://www.biodic.go.jp/bch/download/lmo/R1.5.31_iyaku_ap1.pdf), 3. Method for preparing genetically modified organisms, (2) Method for transferring nucleic acid transferred into host, and this sequence corresponds to the nucleotide numbers 145099 to 145806 of JN555585.1, and the deleted part corresponds to the nucleotide numbers 145328 to 145638 of JN555585.1. The part of this sequence differs from JN555585.1 (one location) is indicated with a box (CCT in JN555585.1, CT in this sequence).

### [Experiment 1]

Expression plasmids (pcDNA3.1+) of the cDNA constructs encoding the exogenous polypeptides were each transfected into HEK293T cells, and after 48 hours, the culture supernatant was collected and concentrated by ultrafiltration using Amicon-Ultra30K. Then, the expression levels of the spike protein and the expression levels of ACE2-binding protein were determined. The former was measured by Spike RBD (receptor-binding domain) detection ELISA (anti-spikeRBD antibody was fixed on a solid phase, and detection was performed with anti-spikeRBD antibody), and the latter by ACE2 binding detection ELISA (ACE2 was fixed on a solid phase, and detection was performed with anti-spikeRBD antibody). The values were calculated as the concentrations in the concentrated solutions.

The results are shown in the following table and in Fig. 2. A: Cov2SPP showed a large expression amount of the spike protein but a low binding ability to ACE2, and B: Cov2SPPF showed a small expression amount of the spike protein and a lower binding ability to ACE2 than C. C: Cov2STH showed a small expression amount of the spike protein but a high binding ability to ACE2, and D: Cov2S1Fc showed a large expression amount of the spike protein and a rather high binding ability to ACE2 (likely to be lower than C).

**[Table 2]**

| | | Spike RBD (ng/ml) | ACE2 bind (ng/ml) | ACE2 bind/ Spike RBD |
|---|---|---|---|---|
| A | Cov2SPP | 12450 | 92 | 0.007 |
| B | Cov2SPPF | 92 | 7 | 0.076 |
| C | Cov2STH | 88 | 202 | 2.304 |
| D | Cov2S1Fc | 4164 | 1391 | 0.334 |

### [Experiment 2]

Vero cells were infected with each virus at MOI 0.01, the culture supernatant was collected after 48 hours and concentrated by ultrafiltration using Amicon-Ultra 30K, and then the expression levels of spike protein and ACE2-binding protein were determined. The former was measured by Spike RBD detection ELISA (anti-spikeRBD antibody was fixed on a solid phase, and detection was performed with anti-spikeRBD antibody) and the latter by ACE2 binding detection ELISA (ACE2 was fixed on a solid phase, and detection was performed with anti-spikeRBD antibody). The values were calculated as the concentrations in the concentrated solutions.

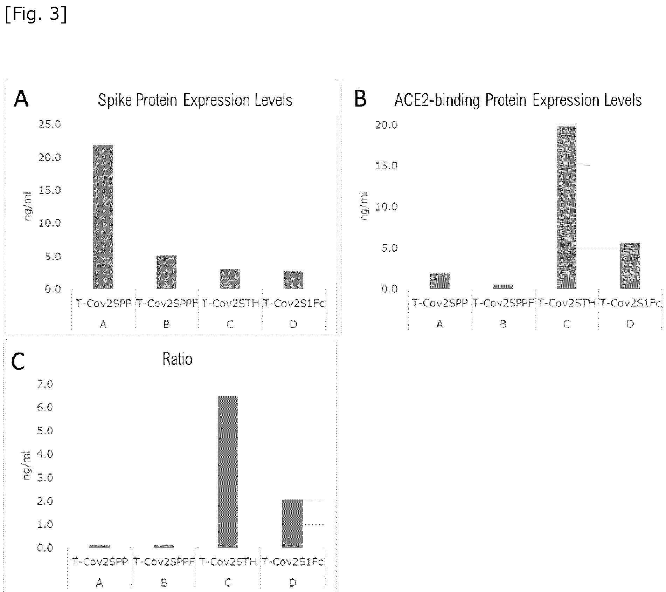

The results are shown in the following table and Fig. 3. In the case of A, the expression amount of the spike protein was large but the binding ability to ACE2 was rather low, and in the case of B, the expression amount of the spike protein was small and the binding ability to ACE2 was also low. In the case of C, the expression amount of the spike protein was small but the binding ability to ACE2 was high, and in the case of D, the expression amount of the spike protein was small but the binding ability to ACE2 was rather high (lower than C, but higher than A and B).

**[Table 3]**

| | | Spike RBD (ng/ml) | ACE2 bind (ng/ml) | ACE2 bind/ Spike RBD |
|---|---|---|---|---|
| A | T-Cov2SPP | 21.9 | 2.0 | 0.090 |
| B | T-Cov2SPPF | 5.2 | 0.5 | 0.094 |
| C | T-Cov2STH | 3.0 | 19.8 | 6.502 |
| D | T-Cov2S1Fc | 2.7 | 5.6 | 2.072 |

### [Experiment 3: In vivo experiment]

Each virus (Benzonaze-treated virus, virus obtained by subjecting collected virus to an enzyme treatment with Benzonase to remove DNA and RNA derived from Vero cells used as the host cells during the virus production) was prepared at 1 x 10⁷ pfu or 1 x 10⁶ pfu/100 µl, and intramuscularly administered to 6-week old female A/J mice at the anterior thighs of hind legs with a 27G needle (100 µl per mouse, which was administered as divided portions into two legs (50 µl per leg)). After 4 weeks (4w), the virus was administered once again. The serum was collected over time after 2, 4, 6, and 8 weeks (see the following table), and the expression levels of anti-Cov2-spike protein antibody was quantified by ELISA (Cov2-spike protein was fixed on a solid phase, and detection was performed with HRP-labeled anti-mouse IgG antibody).

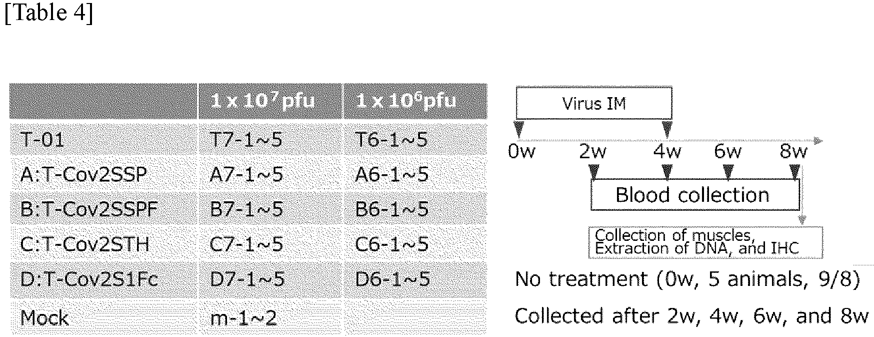

After 8 weeks, muscles were corrected from the administration sites, and the presence or absence of viral DNA was verified by real-time PCR or immunohistochemical staining (IHC). Body weights were also measured before and one day after the administration, and every other week for 8 weeks.

### 1) Expression levels of anti-Cov2spike protein antibody

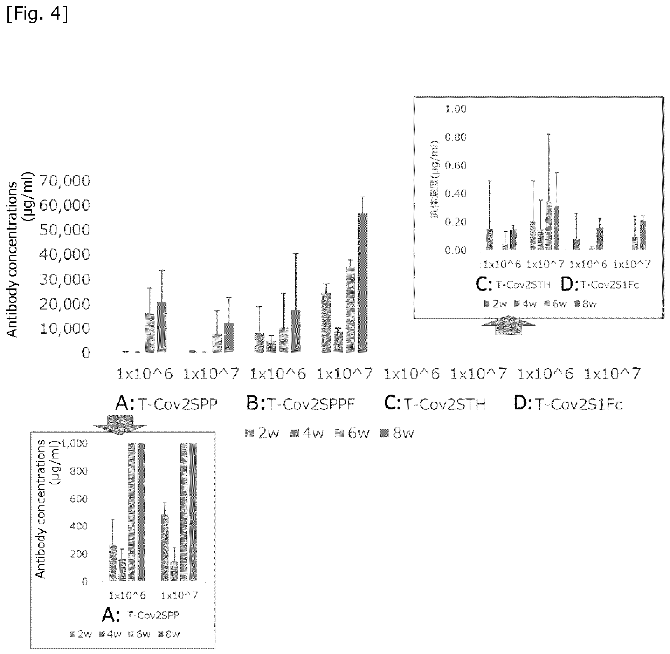

The results for the expression levels of the anti-Cov2spike protein antibody are shown in Fig. 4. The antibody expression levels were in the order of B>A>>C>D. In the case of B, administration of 1 x 10⁷ pfu was more effective, and in the case of A, administration of 1 x 10⁷ pfu provided higher antibody expression levels up to 2 weeks after the administration, but then administration of 1 x 10⁶ pfu provided higher antibody expression levels. In the both cases of A and B, the expression levels tended to decrease from 2 weeks to 4 weeks after the first administration, but the booster administration at 4 weeks markedly increased the antibody expression levels after 6 weeks and 8 weeks. In the cases of C and D, antibody production was detected only in 1 or 2 animals out of 5, and was lower than the detection limit in the remainder.

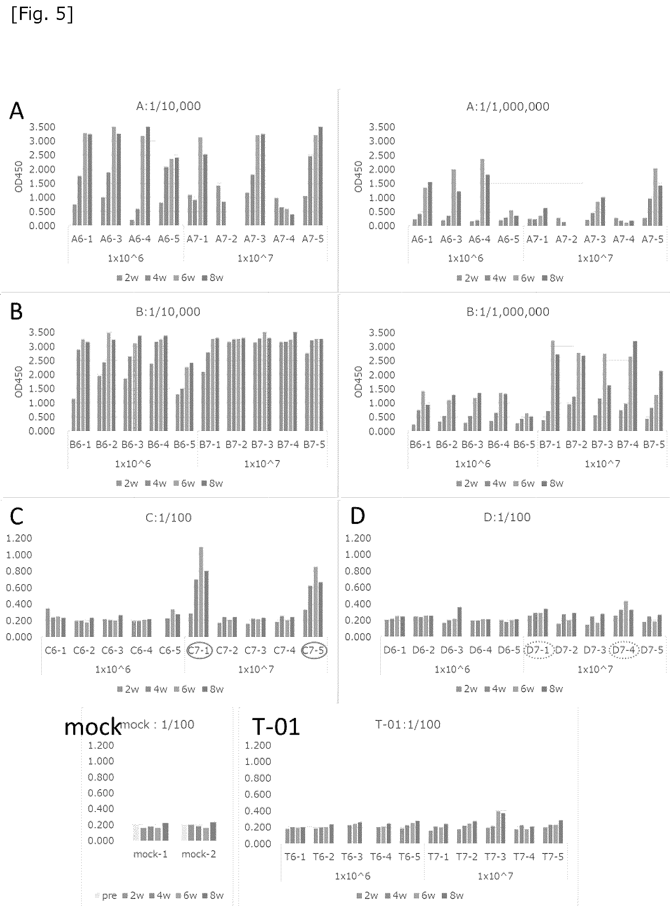

Fig. 5 shows the changes in antibody production amount of individual mice over time. In this figure, the results are shown as OD values measured in ELISA, and the measurement limit was OD=3. A7-2 died 4 weeks after the administration. In the cases of the viruses of A, C, and D, the results significantly varied among the individuals, while relatively stable results were obtained in B. In the case of C, C7-1 and 5 were positive, and in the case of D, D7-1 and 4 were positive. A false positive was observed for T-01 (T7-3).

### 2) Residual amount of virus in muscles after 8 weeks (real-time PCR)

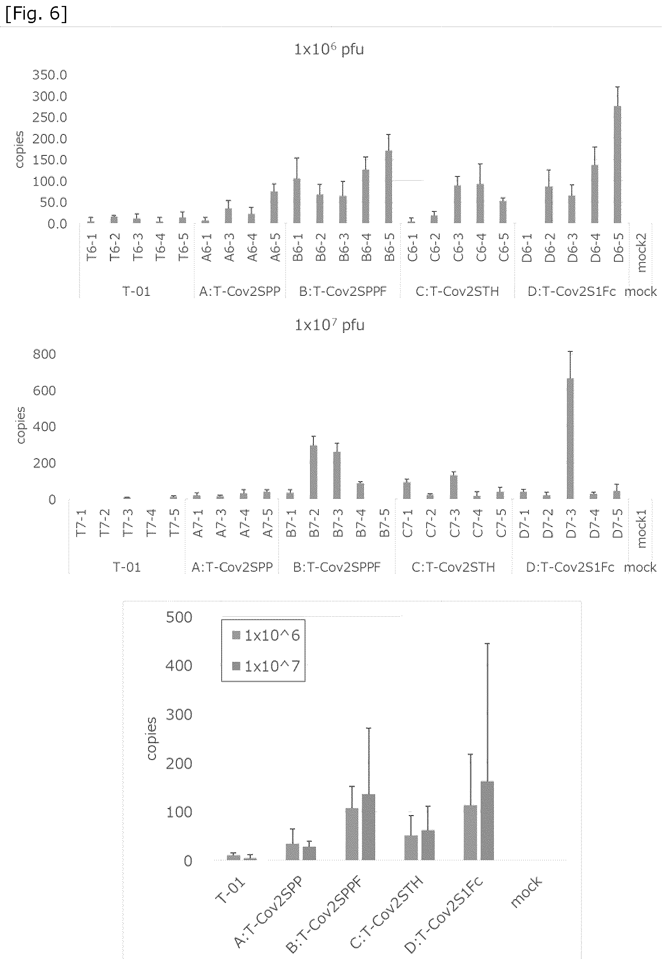

Genomic DNA was prepared from the muscle at the administration site, then real-time PCR was performed by using the ICP6/LacZ probe and primer set, and the copy number of the virus genome was calculated. The results are shown in Fig. 6.

### 3) Pathological image after 8 weeks (HE, and IHC (HSV1, CD4 and CD8))

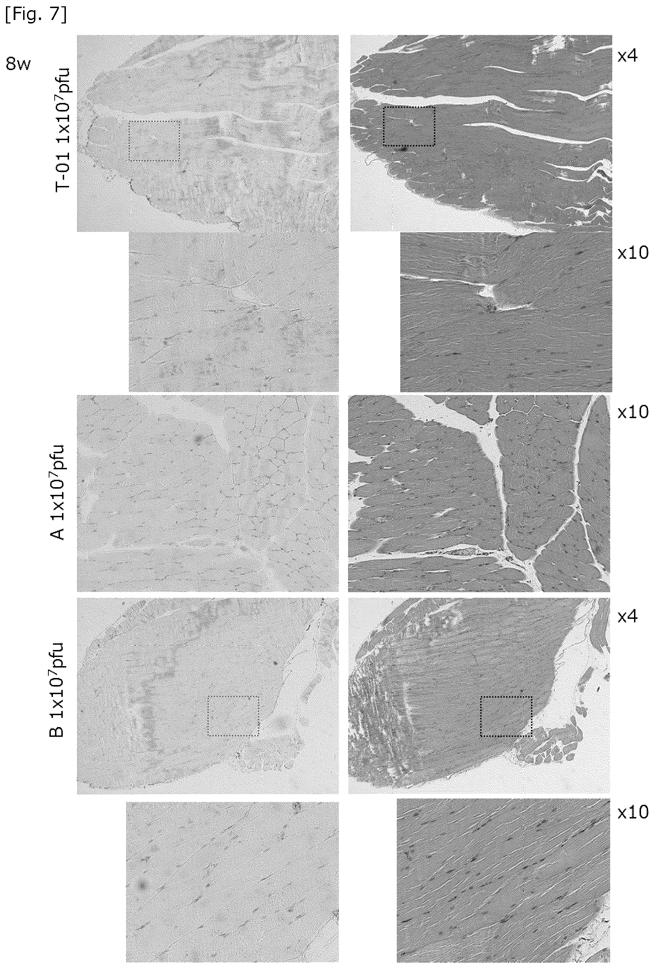

Muscles were collected after 8 weeks, and subjected to HE staining and IHC using anti-HSV-1 antibody (green), anti-CD8 antibody (brown), and anti-CD4 antibody (red). Fig. 7 shows the results for the dose of 1 x 10⁷ pfu as a typical dose, while the dose of 1 x 10⁶ pfu gave substantially the same results. No staining was observed with all of the anti-HSV-1, anti-CD4, and anti-CD8 antibodies.

### 4) Weight change

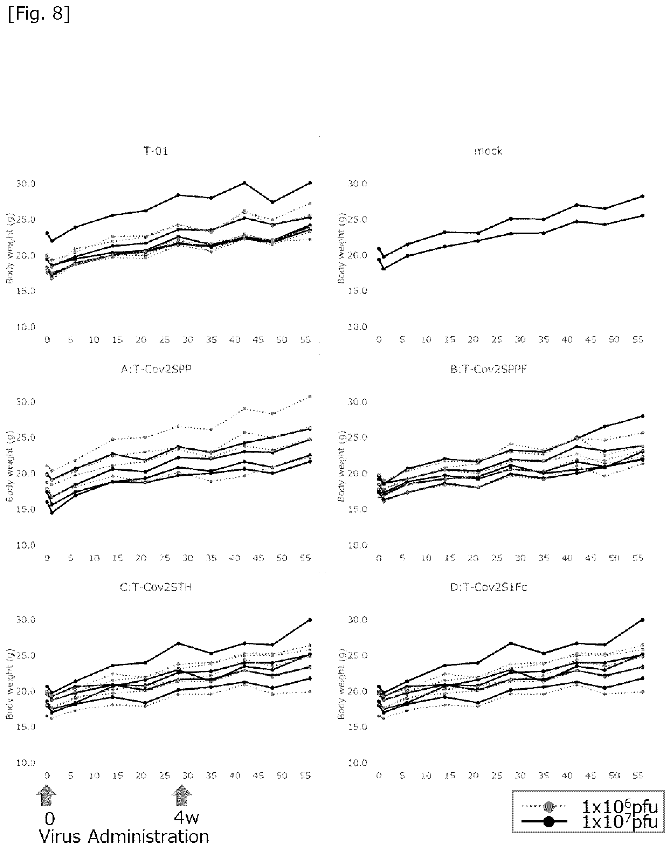

Fig. 8 shows the changes in body weight of individual mice over time. A: in the case of T-Cov2SPP, one mouse died due to anesthesia after each of the administration of 1 x 10⁶ pfu and administration of 1 x 10⁷ pfu. At the time points of 0, 2, 4, 6, and 8 weeks, body weight was measured under anesthesia for virus administration or blood collection. Body weights appeared to be lower when the animals were not anesthetized. The animals showed no abnormal behavior or fur during the observation period. No mice walked strangely.

### [Experiment 3 (supplemental experiment): In vivo experiment, with what kind of temporal change in amount, antibody is produced up to 2 weeks (~ 3 weeks)]

B: T-Cov2SPPF virus was prepared at 1 x 10⁶ pfu or 1 x 10⁷ pfu/100 µl and intramuscularly administered to 6-week old female A/J mice at the anterior thighs of hind legs (100 µl per mouse, which was administered as divided portions into two legs). Serum was collected over time after 6, 12, and 18 days, and the expression levels of anti- Cov2-spike protein antibody was quantified by ELISA (Cov2-spike protein were fixed on a solid phase, and detection was performed with HRP-labeled anti-mouse IgG antibody).

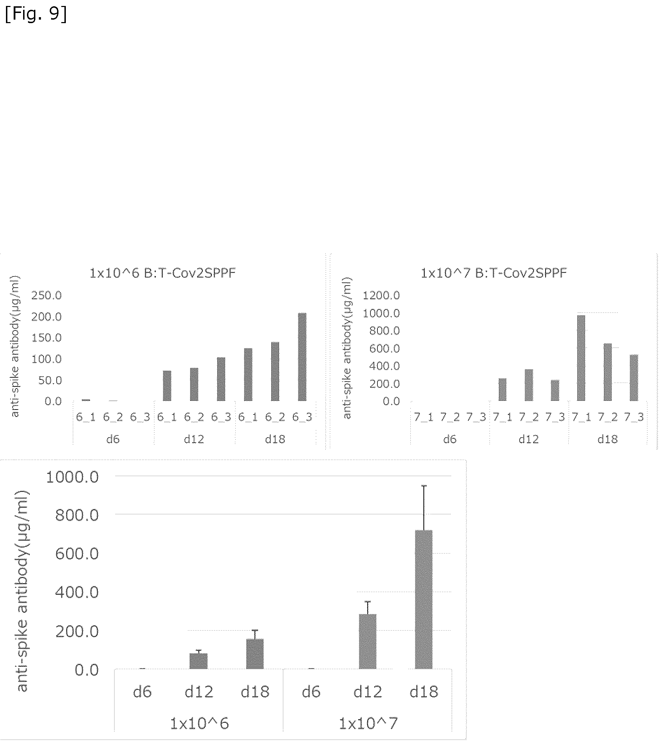

The results are shown in the following table and Fig. 9. Even 6 days after the administration, anti-Cov2-spike protein antibodies were detected and increased over time until day 18.

**[Table 5]**

| Antibody production amount | | |
|---|---|---|
| 1x10^6 | d6 | 2.6 ± 2.1 |
| | d12 | 85.4 ± 16.1 |
| | d18 | 157.9 ± 44.6 |
| 1x10^7 | d6 | 3.0 ± 0.4 |
| | d12 | 287.4 ± 65.4 |
| | d18 | 720.6 ± 230.9 |

### [Experiment 4: In vivo experiment]

Each virus was prepared at 1 x 10⁷ pfu/100 µl and intramuscularly administered to 6-week old or 19-week old female A/J mice at the anterior thighs of hind legs (100 µl per mouse, which was administered as divided portions into two legs). Muscles and serum were collected after 0, 1, 2, and 3 days, and the expression amounts of Cov2-spike protein in muscle and serum were quantified by ELISA (anti-spikeRBD antibody was fixed on a solid phase, and detection was performed with HRP-labeled anti-spikeRBD antibody), and the amount of virus in muscle was quantified by real-time PCR. As the virus, A: T-Cov2SPP, T-01 and mock were used. The virus of A was used because it showed the highest expression amount among the viruses of A to D.

### 1) Expression levels of Cov2-spike protein

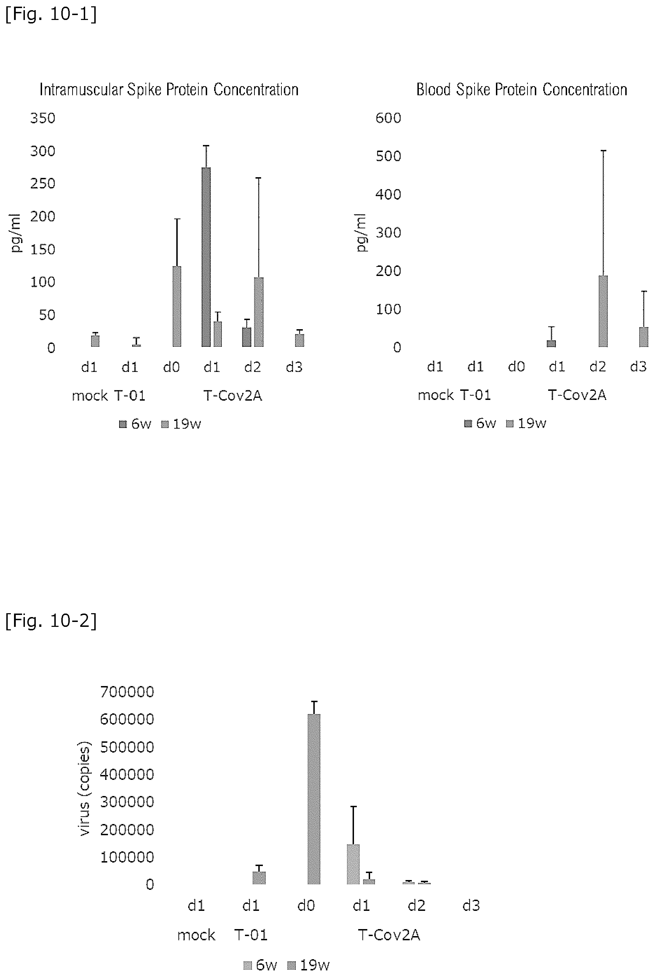

The results are shown in Fig. 10-1. Expression was observed in muscles. It was found that the expression levels were higher in the young mice (6-week old) than in old mice (19-week old). The peak of the expression was on the first day of the administration and decreased with time.

### 2) Time course for viral DNA levels in muscle

The results are shown in the following table and Fig. 10-2. A decrease in the viral DNA levels was observed over time from immediately after the administration. The amount of retained viral DNA tended to be higher in the young mice (6-week old) than in the old mice (19-week old) on both day 1 (7-fold) and day 2 (1.4-fold). The slightly higher viral DNA levels in T-01 on day 1 compared with A was due to the higher titer (actual titer) confirmed after the administration.

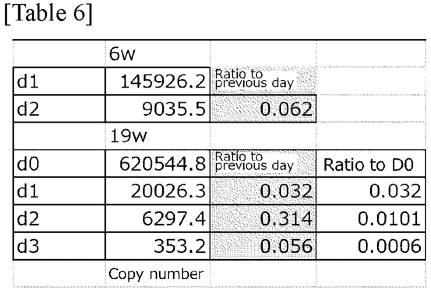

### [Experiment 4 (supplemental experiment): In vivo experiment -Whether virus is detected in blood]

B: T-Cov2SPPF virus was prepared at 1 x 10⁶ pfu or 1 x 10⁷ pfu/100 µl and intramuscularly administered to 6-week old or 17-week old female A/J mice at the anterior thighs of hind legs (100 µl per mouse, which was administered as divided portions into two legs). Muscles and whole blood were collected after 1 and 4 days, and the virus amounts in blood and muscle were quantified by real-time PCR (the virus of B was used because it showed the highest antibody production amount).

### (1) Viral DNA levels in blood

Not detected in all the samples.

### (2) Time course for viral DNA levels in muscle

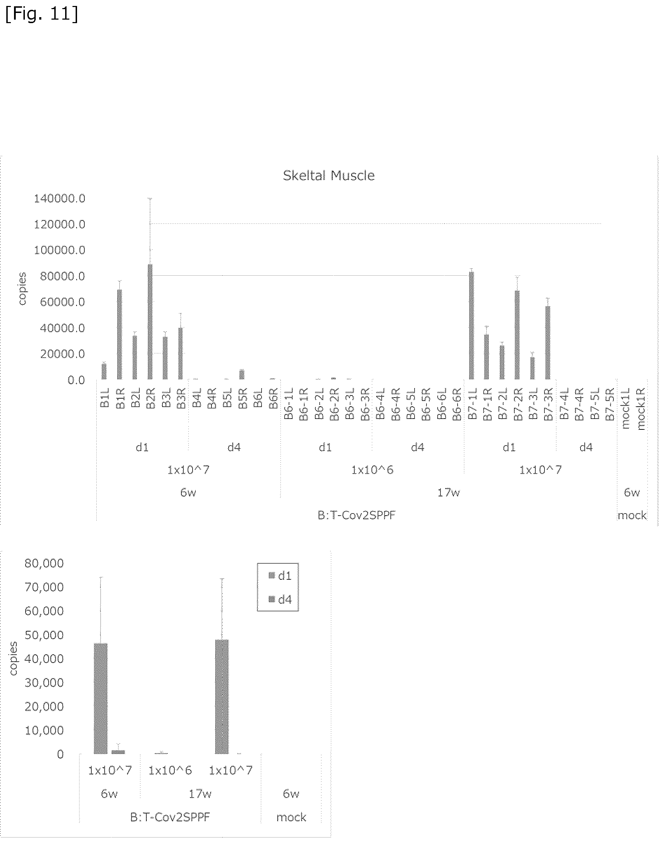

The results are shown in the following table and Fig. 11. The amount of viral in the muscle at the administration site decreased over time. The amount of retained viral tended to be higher in the young mice (6-week old), i.e., 30-fold higher, than in the old mice (17-week old) on day 4.

**[Table 7]**

| | | | d1 | d4 |
|---|---|---|---|---|
| B:T-Cov2SPPF | 6w | 1x10^7 | 46311±27759 | 1557±2734 |
| | 17w | 1x10^6 | 510±465 | 18±8 |
| | | 1x10^7 | 47875±25677 | 52±67 |

### [Experiment 5: In vivo experiment]

Each virus was prepared at 1 x 10⁷ pfu or 1 x 10⁶ pfu*/100 µl and intramuscularly administered to 7-week old female A/J mice at the anterior thighs of hind legs (50 µl per mouse, which was administered as divided portions into two legs). Muscles were collected after 0, 1, and 5 days and subjected to HE staining and IHC using anti-HSV-1 antibody (green), anti-CB8 antibody (brown), and anti-CD4 antibody (red). A: T-Cov2SPP, T-01 and mock were used as viruses.

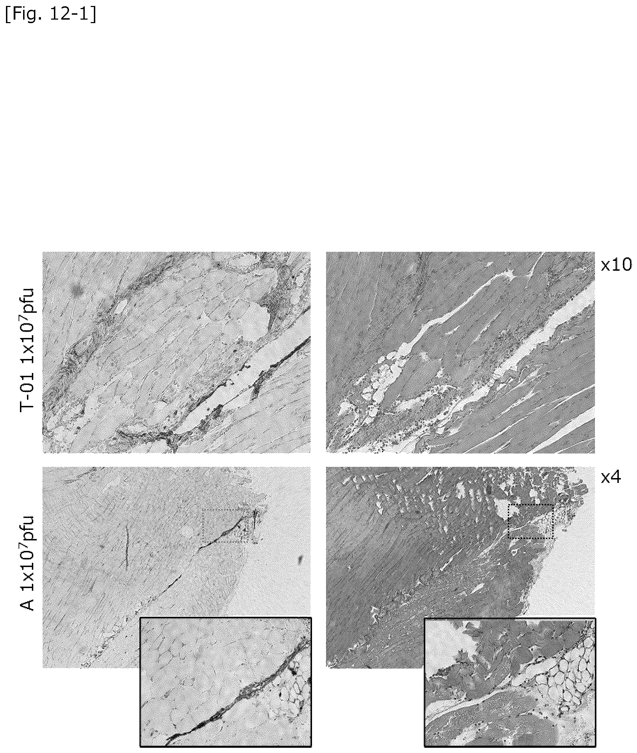

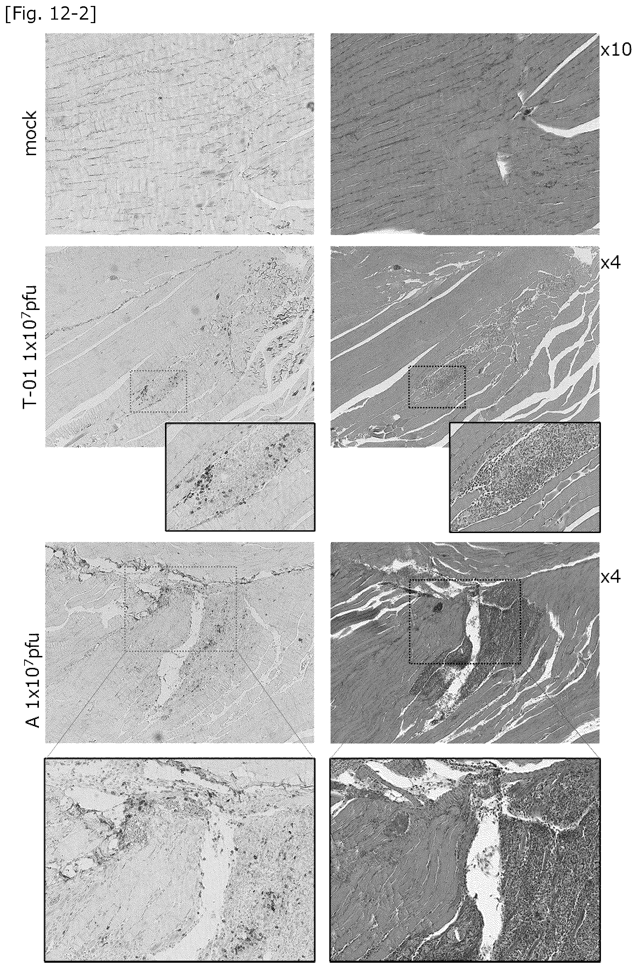

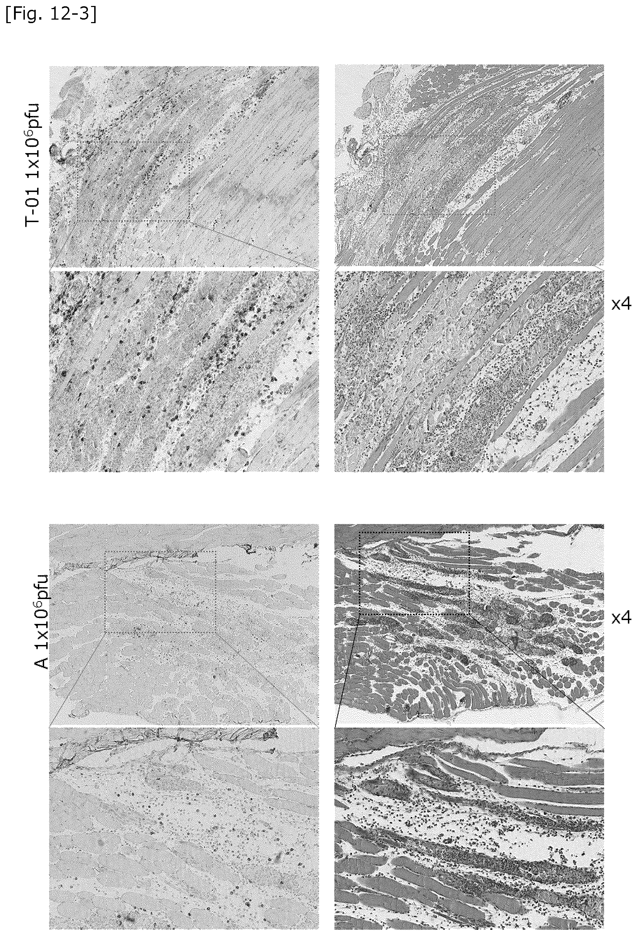

Fig. 12-1 shows the results for the administration of 1 x 10⁷ pfu on day 1. Substantially the same results were also obtained with 1 x 10⁶ pfu. On day 1, HSV-1-positive cells were observed along the administration site for both T-01 and A: T-Cov2SPP. The results obtained with the 1 x 10⁷ pfu administration on day 5 are shown in Fig. 12-2, and the results obtained with the 1 x 10⁶ pfu administration on day 5 are shown in Fig. 12-3. On day 5, many CD8 and CD4-positive cells were observed, possibly due to inflammation at the administration site. Almost no HSV-1-positive cell was observed.

### [Experiment 6: In vivo experiment]

B: T-Cov2SPPF virus was prepared at 1 x 10⁶ pfu/100 µl and intramuscularly administered to 7- to 8-week old female DBA/2 mice at the anterior thighs of hind legs (100 µl per mouse, which was administered as divided portions into two legs), and the following measurements were performed.

1) Muscles were collected after 1, 2, 3, and 4 weeks, and the amount of virus in the muscles were quantified by real-time PCR.
2) Serum was collected after 1, 2, 3, and 4 weeks, and the expression levels of anti-Cov2-spike protein antibody was quantified by ELISA (Cov2-spike protein was fixed on a solid phase, and detection was performed with HRP-labeled anti-mouse IgG antibody).

In addition, B: T-Cov2SPPF virus was administered at 2 x 10⁵ pfu/20 µl into tumors of carcinoma-bearing mice having subcutaneous tumors formed by subcutaneous transplantation of clone M3 cell strain (melanoma) in DBA/2 mice, serum was collected 3 weeks later, and the expression levels of anti-Cov2-spike protein antibody was quantified by ELISA as described above.

The results are shown in Figs. 13-1, 13-2 and the following table.

When B: T-Cov2SPPF virus was intramuscularly administered, only traces of viral DNA were detected in the muscles even after 4 weeks (Fig. 13-1 and the following table).

**[Table 8]**

| | | 1w | 2w | 3w | 4w |
|---|---|---|---|---|---|
| B:T-Cov2SPPF | | 68.0±40.0 | 55.6±13.4 | 45.0±24.3 | 55.7±39.9 |
| mock | | 0.3±0.4 | 0.0±0.0 | 0±0 | 0±0.0 |

Both the A/J and DBA/2 mice are strains relatively susceptible to HSV-1, but the vaccine effect was similarly obtained in different mouse strains (Fig. 13-1, left). The intratumoral (IT) administration resulted in a 35-fold higher blood antibody concentration compared with the intramuscular (IM) administration over the same 3 weeks period, even though the dose was 1/5 of the dose for IM (Fig. 13-2, right, and the following table).

**[Table 9]**

| | | µg/ml |
|---|---|---|
| 1M | 1w | 0.5±0.4 |
| | 2w | 16.4±9.3 |
| | 3w | 24.1±9.3 |
| | 4w | 5.3±3.1 |
| IT | 3w | 834.6±547.4 |

### References, etc.

For more detailed information on the experiments described in the section of Examples, materials and experimental methods described in known references, such as Patent documents 1 to 4 and Non-patent documents 1 to 4 mentioned above, can be referred to.

### Industrial applicability

The present invention provides a novel virus vector. This virus vector can be used as a vaccine, and has potential for use in industrially important fields such as pharmaceuticals (including products for regenerative medicine etc.), research and development, drug manufacturing, and medicine.

### [Sequences listed in Sequence Listing]

SEQ ID NO: 1, Spike protein (GenBank: QHD43416.1), PP (K986P, V987P) mutation
SEQ ID NO: 2, Spike protein (GenBank: QHD43416.1), Furin (R683A R685A), PP (K986P, V987P) mutation
SEQ ID NO: 3, Spike protein (GenBank: QHD43416.1), Furin (R683A R685A), PP (K986P, V987P), mu-phosphatase signal peptide (1..20), fold on trimerization motif (1219..1249), hexa-histidine tag (1250..1256)
SEQ ID NO: 4, Spike S1 (1..685) (GenBank: QIC53204.1, Met1..Arg685), Fc region of human IgG1 (686..917) (GenBank: CAR58103.1, Glu98..Lys329)
SEQ ID NO: 5, Sequence containing LacZ-fused T-Cov2 spike(A) gene insertion site
SEQ ID NO: 6, Sequence containing γ34.5 gene deletion site (TR_{L} region)
SEQ ID NO: 7, Sequence containing α47 gene deletion site (TR_{L} region)
SEQ ID NO: 8, Furin cleavage site

This application claims conventional priority based on Japanese Patent Application No. 2022-129775, filed on August 16, 2022, of which entire disclosure is incorporated herein by reference.

## Claims

1. A pharmaceutical composition for producing an exogenous polypeptide and inducing an immune response thereto, the composition comprising a herpes simplex virus vector containing a region encoding the exogenous polypeptide and having at least one characteristic selected from the following characteristics (a) to (c):
(a) the ICP6 gene is deleted or inactivated,
(b) the γ34.5 gene is deleted or inactivated, and
(c) the α47 gene is deleted or inactivated.

2. The pharmaceutical composition according to claim 1, wherein the herpes simplex virus vector has all the characteristics (a) to (c).

3. The pharmaceutical composition according to claim 2, wherein the region encoding the exogenous polypeptide is inserted into a deleted or inactivated site of the ICP6 gene.

4. The pharmaceutical composition according to any one of claims 1 to 3, which is for producing the exogenous polypeptide in noncancerous cells.

5. The pharmaceutical composition according to any one of claims 1 to 4, which is an agent for subcutaneous or intramuscular injection.

6. The pharmaceutical composition according to any one of claims 1 to 3,
wherein the exogenous polypeptide contains all or a part of a pathogen-derived protein.

7. The pharmaceutical composition according to claim 6, wherein the exogenous polypeptide contains all or a part of a spike protein derived from SARS-CoV-2.
